# EUROPÄISCHE PATENTSCHRIFT

(11) **EP 2 292 224 B1**
(45) Veröffentlichungstag und Bekanntmachung des Hinweises auf die Patenterteilung: **07.03.2018**
(21) Anmeldenummer: 10169426.3
(22) Anmeldetag: 13.07.2010
(51) Int. Cl.: A61K 31/216, A61K 31/25, A61P 3/10

(54) **Lactisolverbindungen zur Prävention oder Behandlung von Diabetes**
Derivatives of lactiosole for preventing or treating diabetes
Dérivés du lactisole pour la prévention ou le traitement du diabète

(30) Priorität: 15.07.2009 DE 102009027744
(43) Veröffentlichungstag der Anmeldung: 09.03.2011
(73) Patentinhaber: Symrise AG, 37603 Holzminden (DE); Deutsches Institut für Ernährungsforschung Potsdam-Rehbrücke, 14558 Nuthetal (DE)
(72) Erfinder: Ley, Jakob Peter, Dr., 37603 Holzminden (DE); Backes, Michael, Dr., 37603 Holzminden (DE); Vössing, Tobias, 37688 Beverungen (DE); Stähler, Frauke, Dr., 13089 Berlin (DE); Meyerhof, Wolfgang, Prof. Dr., 22848 Norderstedt (DE); Wintermeyer, Christian, 37671 Höxter (DE)
(74) Vertreter: Fabry, Bernd

(56) Entgegenhaltungen:
- EP-A2- 0 159 864
- WO-A2-2009/026389
- US-A- 3 494 957
- DATABASE WPI Week 201027 Thomson Scientific, London, GB; AN 2010-D75022 XP002603817 LI D.; LI X.;XU Z.: "Novel Phenoxy Acetic Acid Pyrazine Ester DerivativeUseful in Pharmaceutical Composition for Reducing Blood lipid, for Reducing Serum Triglyceride level and Serum Cholesterol and for Elevating High-density Lipoprotein" -& WO 2010/034212 A (ANHUI NEW STAR PHARM DEV CO LTD) 1. April 2010 (2010-04-01)
- HEATHCOCK, CLAYTON H. ET AL: "Acyclic stereoselection. 23. Lactaldehyde enolate equivalents" JOURNAL OF THE AMERICAN CHEMICAL SOCIETY, Bd. 106, Nr. 26, 1984, Seiten 8161-8174, XP002603815
- CAMPS P ET AL: "(R)- and (S)-3-Hydroxy-4,4-dimethyl-1-phenyl-2-pyrr olidinone as chiral auxiliaries for the asymmetric synthesis of alpha-hydroxy acids" TETRAHEDRON ASYMMETRY, Bd. 8, Nr. 11, 12. Juni 1997 (1997-06-12), Seiten 1877-1894, XP004074674 PERGAMON PRESS LTD, OXFORD, GB ISSN: 0957-4166 DOI: 10.1016/S0957-4166(97)00176-6
- JIANG PEIHUA ET AL: "Lactisole interacts with the transmembrane domains of human T1R3 to inhibit sweet taste." THE JOURNAL OF BIOLOGICAL CHEMISTRY, Bd. 280, Nr. 15, 15. April 2005 (2005-04-15), Seiten 15238-15246, XP002603816 ISSN: 0021-9258

## Beschreibung

### GEBIET DER ERFINDUNG

Die vorliegende Erfindung betrifft spezielle Lactisolverbindungen zur Prävention bzw. Behandlung von Diabetes, Zusammensetzungen, die diese Stoffe enthalten sowie die Verwendung der Stoffe als Süßrezeptor-antagonisten.

### STAND DER TECHNIK

Es ist seit langem bekannt, dass eine erhöhte Kalorienaufnahme, insbesondere eine erhöhte Kalorienaufnahme in Form von süß schmeckenden Zuckern oder leicht spaltbaren Kohlenhydraten, die im Körper rasch Glucose freisetzen, wesentlich zur Erhöhung der Diabetes-Inzidenz beiträgt, im speziellen zur Inzidenz von Diabetes Typ 2 (Diabetes mellitus Typ 2, früher als "Alters-Diabetes" bezeichnet). Bei Diabetes Typ 2 liegt ein sogenannter "relativer Insulinmangel" vor. Der Botenstoff Insulin ist zwar vorhanden, kann an seinem Zielort, den Zellmembranen, jedoch nicht bzw. nicht ausreichend wirken, da die Zellen nicht oder nur schwach auf Insulin reagieren (Insulinresistenz). Eine solche Insulinresistenz geht der Manifestation des Diabetes in der Regel mehrere Jahre voraus und kann (neben der genetischen Veranlagung) unter anderem durch ständig erhöhte Kalorienaufnahme verstärkt oder verursacht werden: Infolge eines ständig erhöhten Glucose-Spiegels im Blut und der damit verbundenen dauerhaft erhöhten Insulin-Ausschüttung wird die Insulin-Empfindlichkeit der Zellen durch Herunter-Regulierung der Insulin-Rezeptoren herabgesetzt. Um die verminderte Insulinempfindlichkeit auszugleichen, produziert die Bauchspeicheldrüse immer größere Mengen an Insulin. Nach einem bestimmten Zeitraum kann die Bauchspeicheldrüse diese überhöhte Insulinproduktion - aufgrund dadurch auftretender Erschöpfung und frühzeitigem Zelltod der Insulin-produzierenden Inselzellen im Pankreas - jedoch nicht mehr aufrechterhalten. Die produzierte Insulinmenge reicht dann nicht mehr aus, um den Blutzuckerspiegel (ausreichend) zu kontrollieren und der Diabetes Typ 2 wird manifest.

So wurden in der Vergangenheit nichtkalorische Zuckeraustauschstoffe wie Zuckeralkohle (z.B. Fructose, Erythritol [E968], Isomalt [E953], Isomaltulose, Lactitol [E966], Maltitol [E965], Mannitol [E421], Sorbitol [E420], Trehalose, Xylitol [E967]) oder hochintensive Süßstoffe (z.B. Acesulfam K [E950], Alitam, Aspartam [E951], Brazzein, Cyclamat [E952], Neohesperidine DC [E959], Neotam, Saccharin [E954], Steviosid, Sucralose [E955] als Alternative zu Glucose diskutiert und in einer Vielzahl ehemals zuckerhaltiger Produkte eingesetzt. Diese Stoffe sollen durch ihren (teilweise sehr starken) Süßgeschmack das hedonistische Bedürfnis nach Süße befriedigen ohne den Glucose-Spiegel im Blut (nachteilig) zu erhöhen.

Gymnemasäuren aus der Pflanze *Gymnema sylvestre* werden bereits als Anti-Diabetes-Mittel eingesetzt (Suttisri R, Lee I-S & Kinghorn AD (1995) Plant-derived triterpenoid sweetness inhibitors. J. Ethnopharmacol., 47, 9-26).

Die primären Rezeptoren sowie die zur Signalübertragung ans Gehirn notwendige Signalkaskade in den Geschmackszellen bzw. -knospen zur Süßwahrnehmung im Mund, insbesondere auf der Zunge, sind im Stand der Technik bereits beschrieben (Meyers B & Brewer MS (2008) Sweet taste in man: a review. Journal of Food Science, 73(6), R81-R90). Nach Chandrashekar *et al.* (Chandrashekar J, Hoon MA, Ryba NJP & Zucker CS (2006) the receptors and cells for mammalian taste. Nature, 444(7117), 288) binden sämtliche der bisher untersuchten Süßstoffe an das GPCR-Heterodimer (GPCR: G protein-coupled receptor, G-Protein-gekoppelter Rezeptor) T1R2/T1R3 (Tas1R2, taste receptor, type 1, member 2 / Tas1R3, taste receptor, type 1, member 3). Die Rezeptorbindung bzw. die Signalübertragung kann durch (Süßrezeptor-)Antagonisten wie beispielsweise Lactisol dosisabhängig gehemmt werden (Xu H, Staszewski L, Tang H, Adler E, Zoller M & Li X (2004) Different functional roles of T1R subunits in the heteromeric taste receptors. Proceedings of the National Academy of Sciences of the United States of America, 101, 14258-14263; Jiang, P., M. Cui, et al. (2005). "Lactisole Interacts with the Transmembrane Domains of Human T1R3 to Inhibit Sweet Taste." Journal of Biological Chemistry 280(18): 15238-15246). Am Beispiel von Lactisol wurde dieser Effekt bereits in Geschmackstests dokumentiert (Schiffmann SS, Booth BJ, Sattely-Miller EA, Graham BG & Gibes KM (1999) Selective inhibition of sweetness by the sodium salt of +/-2-(4-methoxyphenoxy)propanoic acid. Chemical Senses, 24(4), 439-447). Details zu dem Süßrezeptor-Antagonisten Lactisol werden weiter unten beschrieben.

Gemäß neuen Erkenntnissen befinden sich die für die Süßrezeption verantwortlichen T1R2/T1R3-Rezeptoren nicht nur auf der Zunge, sondern auch in spezialisierten Zellen des Darmepithels, insbesondere des Dünndarms (Dyer J, Salmon KSH, Zibrik L & Shirazi-Beechey SP (2005) Expression of sweet taste receptors of the T1R family in the intestinal tract and enteroendocrine cells. Biochemical Society Transactions, 33(1), 302-305). Neben dem Heterodimer T1R2/T1R3 wurde auch die gesamte für den Süßgeschmack bekannte Signalkaskade (alpha-Gustducin, Phospholipase-C-beta 2, TRPM5) in den "Solitary Intestinal Epithelial Cells" des Dünndarms im Menschen gefunden (Bezençon C, le Coutre J & Damak S (2007) Taste-Signaling Proteins Are Coexpressed in Solitary Intestinal Epithelial Cells. Chem. Sens., 32(1), 41-49). Es ist zudem bekannt, dass (zumindest in Ratten) von diesen Zellen auch Hormone abgesondert werden, die unter anderem die Glucose-Aufnahme aus dem Darm mittels GLUT-2 (glucose transporter isoform 2) oder über den Regulator SGLT1 (sodiumdependent glucose transporter isoform 1) fördern, d.h. den Blutzuckerspiegel indirekt erhöhen, können (Mace OJ, Affleck J, Patel N & Kellett GL (2007) Sweet taste receptors in rat small intestine stimulate glucose absorption through apical GLUT2. Journal of Physiology, 582(1), 379-392 und Margolskee RF, Dyer J, Kokrashvili Z, Salmon KSH, Ilegems E, Daly K, Maillet EL, Ninomiya Y, Mosinger B & Shirazi-Beechey SP (2007) T1R3 and gustducin in gut sense sugars to regulate expression of Na+-glucose cotransporter 1. Proceedings of the National Academy of Sciences of the United States of America, 104(38), 15075-15080).

Nach bisherigem Kenntnisstand unterscheiden sich die Süßrezeptoren in der Zunge hinsichtlich ihrer Selektivität nicht von denen im (Dünn-)Darm. Zwar bedeutet dies, dass Süßrezeptor-Antagonisten wie z.B. Lactisol, Gurmarin oder Gymnemasäure (siehe unten, WO 2009 026389 A2**,** MOUNT SINAI) in der Regel sowohl die Süßrezeptoren im Mundraum als auch die Süßrezeptoren im Darm inhibieren (können). Jedoch eignen sich solche Süßrezeptor-Antagonisten für die Zwecke der Inhibierung von Süßrezeptoren im Darm eben aufgrund ihrer geschmacksmodifizierenden Wirkung, nämlich der Reduzierung des süßen Geschmackseindrucks im Mundraum, weniger für den Einsatz in Nahrungsmitteln, insbesondere nicht für den Einsatz in primär süß schmeckenden Nahrungsmitteln.

In der oben genannten Schrift werden Methoden offenbart, die unter Verwendung von "sweet taste inhibitors" (wie z.B. Lactisol, Gurmarin, Gymnemasäure, substituierten Sulfamaten und substituierten Cyclamat-Sulfamaten) oder "sweet taste potentiators" eine Beeinflussung (eine Verstärkung oder eine Inhibierung) der Süßrezeptoren im Darm ermöglichen. Insoweit wird auch eine Maßnahme beschrieben, um (bei oraler Applikation) die Beeinflussung der Geschmackszellen im Mundraum zu verhindern. Hierfür werden die Süßrezeptor-Antagonisten in einen pharmazeutischen Träger eingekapselt, der die Süßrezeptor-Antagonisten im Magen, im Dünn- oder im Dickdarm freisetzt, wobei der pharmazeutische Träger so gestaltet sein kann, dass ein Kontakt der Süßrezeptor-Antagonisten mit den Geschmackszellen im Mundraum im Wesentlichen verhindert wird. In WO 2009/026389 A2 werden jedoch keine Prekursoren (Lactisolverbindungen) von Süßrezeptor-Antagonisten, insbesondere keine Lactisolverbindungen von Lactisol, beschrieben, die durch die körpereigene Verdauung erst im Darm in einen (aktiven) Süßrezeptor-Antagonisten sowie gegebenenfalls weitere zur Verwendung in Lebensmitteln zugelassene bzw. geeignete Verbindungen metabolisiert werden.

Primäre Aufgabe der vorliegenden Erfindung war es, pharmazeutische Formulierungen oder Stoffe (Verbindungen) bereitzustellen, bei deren Verwendung selektiv die Süßrezeptoren im Darm, insbesondere im Dünndarm, inhibiert werden können, ohne die Süßrezeptoren im Mundraum (nachteilig) zu beeinflussen. Insbesondere galt es dabei pharmazeutische Formulierungen oder Stoffe zu finden, die idealerweise (nahezu) geschmacklos sind und vorzugsweise auch den Geschmackseindruck anderer Stoffe im Mundraum nicht beeinflussen. Bevorzugt sollten Stoffe bereitgestellt werden, die sich besonders gut zur direkten Verarbeitung in Lebensmitteln eignen und keiner weiteren Formulierung (z.B. magensaftresistente Verkapselung) bedürfen.

Insbesondere sollten solche pharmazeutische Formulierungen oder Substanzen bereitgestellt werden, die sich zur Prävention oder Behandlung von Diabetes, insbesondere von Diabetes Typ 2, eignen.

Eine weitere Aufgabe der vorliegenden Erfindung war es, entsprechende zum Verzehr geeignete Zusammensetzungen, der Ernährung oder dem Genuss dienende Zubereitungen sowie pharmazeutische Zubereitungen, insbesondere pharmazeutische Zubereitungen zur oralen Applikation, bereitzustellen.

### GEGENSTAND DER ERFINDUNG

Die vorliegende Erfindung betrifft Lactisol-Verbindungen der Formel (I) 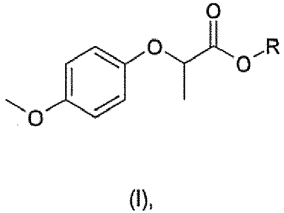 in der R für einen aromatischen oder aliphatischen Rest steht, der eine Gruppe -O- umfasst, die Bestandteil einer Hydroxy-, Ester- oder Ether-Gruppe ist, zur Verwendung als Arzneimittel zur Prävention und/oder Behandlung von Diabetes, speziell von Diabetes Typ-2.

### LACTISOLVERBINDUNGEN

Die Süßrezeptor inhibierende Wirkung von Lactisol wurde bereits weiter oben beschrieben. Lactisol (2-(4-Methoxyphenoxy)propionsäure; CAS-Nummer [13794-15-5]) und diverse Derivate sowie Salze davon sind darüber hinaus schon seit längerem als Geschmacksmodulatoren bekannt. Zum Beispiel wurde das Natriumsalz des Lactisol bei der FEMA (**F**lavor & **E**xtract **M**anufacturers **A**ssociation) unter der Nummer 3773 angemeldet und besitzt seitdem den GRAS-Status (**G**enarally **R**ecognized **A**s **S**afe) für den Einsatz in Lebensmitteln. In EP 159864 wird beispielsweise die Verwendung dieses Natriumsalzes zur Reduzierung der Eigensüße diverser zuckerhaltiger Lebensmittel offenbart.

Des Weiteren wurde Lactisol in gerösteten Arabica Kaffebohnen aus Kolumbien (in Konzentrationen zwischen 0,55 ppm bis 1,2 ppm) nachgewiesen (Rathbone EB, Patel GD, Butters RW, Cookson D, Robinson JL (1989); Occurrence of 2-(4-Methoxyphenoxy)propanoic Acid in Roasted Coffee Beans: Analysis by Gas-Liquid Chromatography and by High-Performance Liquid Chromatography. J. Agric. Food Chem. 37, 54-58). Gemäß einer weiteren Veröffentlichung liegt das darin nachgewiesene Lactisol in überwiegendem Anteil (bis zu etwa 80%) in der (*S*)-konfigurierten Form vor (Rathbone EB, Butters RW, Cookson D, Robinson JL (1989); Chirality of 2-(4-Methoxyphenoxy)propanoic Acid in Roasted Coffee Beans: Analysis of the Methyl Esters by Chiral High-Performance Liquid Chromatography. J. Agric. Food Chem. 37, 58-60).

Im Stand der Technik wurden zudem verschiedene Lactisolester beschrieben. Insbesondere sind Methyl-, Ethyl- und n-Butylester als Synthesebausteine bekannt. Ferner befassen sich diverse Veröffentlichungen mit der Gewinnung von optisch aktivem Lactisol oder entsprechenden Estern davon. Neben einer asymmetrischen Synthese (z.B. Chen C, Zhu SF, Liu B, Wang LX & Zhou QL (2007) Highly Enantioselective Insertion of Carbenoids into O-H Bonds of Phenols: An Efficient Approach to Chiral alpha-Aryloxycarboxylic Esters. J. Am. Chem. Soc. 129(42) 12616-12617) wurde im Stand der Technik auch eine Herstellung durch Racematspaltung beschrieben (z.B. Nishigaki T, Yasufuku Y, Murakami S, Ebara Y & Ueji SI (2008) A Great Improvement of the Enantioselectivity of Lipase-Catalyzed Hydrolysis and Esterification Using Co-Solvents as an Additive. Bull. Chem. Soc. Jpn 81(5) 617-622).

In WO 2004 002925 A2 (KOREA RESEARCH) wird die Synthese von enantiomerenreinen (R)-Aryloxypropansäureestern (z.B. von (R)-Lactisolethylester) ausgehend von (S)-Milchsäuretosylaten beschrieben. Erfindungsgemäße Lactisolverbindungen von Süßrezeptor-Antagonisten zur Prävention oder Behandlung von Krankheiten, insbesondere zur Prävention oder Behandlung von Diabetes (Typ 2), werden jedoch nicht beschrieben.

In EP 0528268 B1 (BASF) wird die Herstellung und Verwendung verschiedener Lactisolester als feste oder flüssigkristalline optisch anisotrope Medien beschrieben. Die alkoholischen Ester-Bestandteile sind dabei überwiegend Biphenyle, wie beispielsweise im folgenden Formelbild gezeigt:

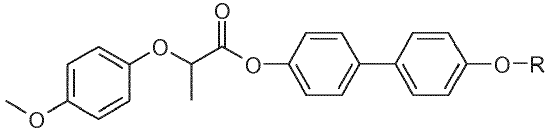

Eine orale Verabreichung der in dieser Schrift beschriebenen Lactisolester ist nicht vorgesehen. Erfindungsgemäße Lactisolverbindungen von Süßrezeptor-Antagonisten zur Prävention oder Behandlung von Krankheiten, insbesondere zur Prävention oder Behandlung von Diabetes (Typ 2), werden in EP 0 528 268 nicht beschrieben.

US 4,687,849 (HOFFMANN LA ROCHE) beschreibt eine Verbindung der folgenden Formel:

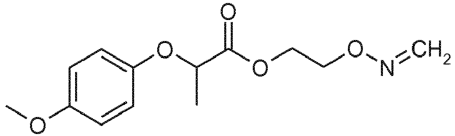

Die enzymatische Hydrolyse von bestimmten Lactisolestern zur Gewinnung der korrespondierenden optisch aktiven Verbindungen wird unter anderem in EP 0214569 A2 (HOECHST) beschrieben.

Erfindungsgemäße Lactisolverbindungen zur Prävention oder Behandlung von Krankheiten, insbesondere zur Prävention oder Behandlung von Diabetes (Typ 2), werden in den Dokumenten jedoch nicht beschrieben.

In den vorangehend genannten Dokumenten wird zudem weder über die sensorischen Eigenschaften der genannten Lactisolester bzw. -derivate noch über deren (medizinische) Wirkung bei oraler Aufnahme berichtet.

In JP 43 018536**,** JP 43 012352 und JP 43 002330 (siehe auch US 3,494,957**,** YOSHITOMI) werden Polyester diverser Lactisolderivate (siehe beispielsweise die folgenden beiden Formelbilder) beschrieben, die zur Senkung des Cholesterinspiegels eingesetzt werden können.

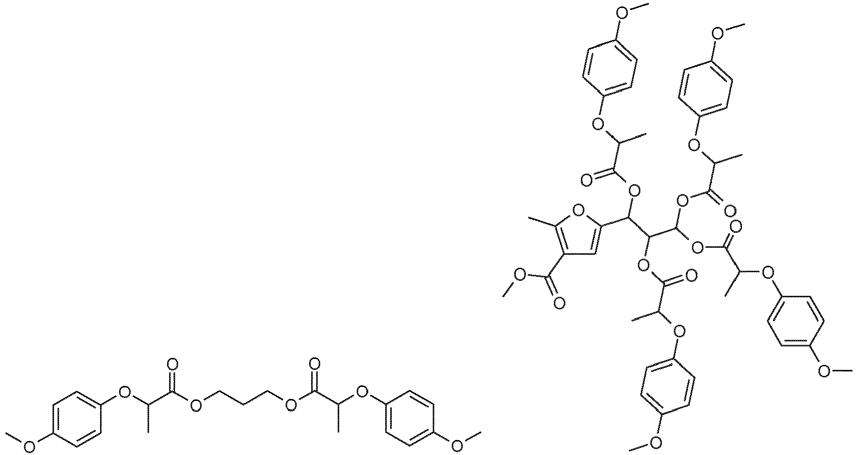

Auf die Verbindungen der beiden vorstehend gezeigten Formelbilder wurde - im Zusammenhang mit einer erfindungsgemäßen Lactisolverbindung bzw. Mischung zur Prävention oder Behandlung von Krankheiten allgemein - bereits weiter oben Bezug genommen (dort als Verbindungen der Formeln (X1) und (X2) bezeichnet).

Die Verbindungen der allgemeinen Formel 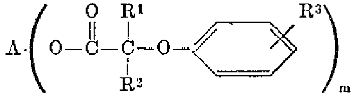 (wie in US 3,494,957 beschrieben), wobei R¹ Methyl, R² Methyl oder Ethyl und R³ H, Cl, Methyl oder Methoxy bedeuten, sind keine (erfindungsgemäßen) Lactisolverbindungen im Sinne der vorliegenden Erfindung.

Weitere im Stand der Technik beschriebene Lactisolester sind die Verbindungen der Formeln 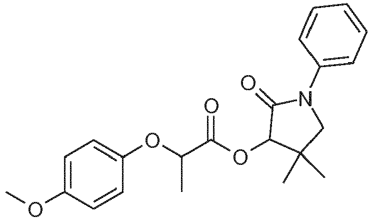 (vgl. Camps P, Perez F, Soldevilla N (1997) Asymmetric synthesis of α-hydroxy acids using (R)- and (S)-3-hydroxy-4,4-dimethyl-1-phenyl-2-pyrrolidinone as chiral auxiliaries. Tetrahedron: Asymmetry 8(11), 1877-1894) und 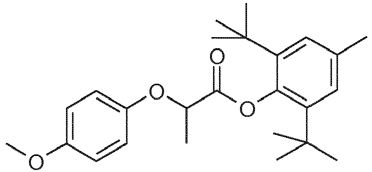 (vgl. Heathcock, CH, Pirrung MC, Young SD, Hagen JP, Jarvi ET, Badertscher U, Marki HP, Montgomery SH (1984) Acyclic stereoselection. 23. Lactaldehyde enolate equivalents. Journal of the American Chemical Society 106(26), 8161-74).

Das chirale Zentrum der Lactisol-Gruppe(n) einer Lactisolverbindung kann jeweils (R)- oder (S)-konfiguriert sein. Eine erfindungsgemäße Mischung, im speziellen eine erfindungsgemäße Mischung zur Prävention oder Behandlung von Diabetes, insbesondere von Diabetes Typ 2, (wie oben beschrieben) umfasst oder besteht dementsprechend vorzugsweise aus verschiedenen Enantiomeren einer oder mehrerer Lactisol-Lactisolverbindungen, wobei die verschiedenen Enantiomere in der Mischung jeweils in einem racemischen, d.h. einem äquimolaren, oder in einem beliebigen anderen Mengenverhältnis zueinander vorliegen.

Erfindungsgemäß besonders bevorzugt ist eine erfindungsgemäße Lactisolverbindung wobei der Rest R der Verbindung der Formel (I) bzw. ein, mehrere oder sämtliche Reste R der unterschiedlichen Verbindungen der Formel (I) unabhängig voneinander eine oder mehrere Gruppen -O- umfasst bzw. umfassen, die jeweils unabhängig voneinander Bestandteil einer Hydroxy-, Ester- oder Ether-Gruppe ist bzw. sind.

Besonders bevorzugt umfasst der Rest R der Verbindung der Formel (I) bzw. ein, mehrere oder sämtliche Reste R der unterschiedlichen Verbindungen der Formel (I) eine oder (vorzugsweise) mehrere Gruppen -O-, die Bestandteil einer Ester-Gruppe ist bzw. sind. Ganz besonders bevorzugt ist über eine solche Ester-Gruppe jeweils eine (weitere) Lactisol-Gruppe (2-(4-Methoxyphenoxy)propionyloxy-) gebunden.

Wie oben beschrieben, sind die erfindungsgemäßen Lactisolverbindungen vorteilhafterweise dazu geeignet, im Darm einen oder mehrere unterschiedliche Süßrezeptor-Antagonisten freizusetzen, vorzugsweise einen oder mehrere unterschiedliche T1R2/T1R3-Rezeptor-Antagonisten (wie z.B. Lactisol). Neben den Süßrezeptor-Antagonisten werden - beispielsweise bei der Spaltung durch Lipasen (wie unten beschrieben) - im (Dünn-)Darm ein oder mehrere weitere Bestandteile, insbesondere Alkohole, freigesetzt (für bevorzugte im Darm freigesetzte weitere Bestandteile siehe unten), wobei die weiteren Bestandteile vorzugsweise für die Verwendung in Lebensmitteln und/oder pharmazeutischen Präparaten zugelassen sind. So wird bei den Lactisolverbindungen der Formel (I) 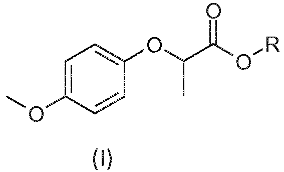 neben Lactisol regelmäßig eine Verbindung R-OH freigesetzt, wobei die Verbindung R-OH eine aliphatische oder aromatische Verbindung mit einer oder mehreren Hydroxy-Gruppen ist.

Bei erfindungsgemäß besonders bevorzugten Verbindungen der Formel (I) mit zwei oder mehr Lactisol-Gruppen (wie oben beschrieben), d.h. bei Verbindungen der Formel (I), bei denen der Rest R eine oder mehrere Ester-Gruppen umfasst, über die eine (weitere) Lactisol-Gruppe gebunden ist, werden regelmäßig (je Molekül einer Verbindung der Formel (I)) zwei oder mehr Moleküle Lactisol freigesetzt.

Die weiteren im (Dünn-)Darm freigesetzten Bestandteile, insbesondere die Verbindungen R-OH, sind vorzugsweise ausgewählt aus der Gruppe bestehend aus folgenden Verbindungen (in den Klammern sind - jeweils sofern vorhanden - die FEMA-Nummer (vierstellig) und die CAS-Nummer oder die E-Nummer angegeben):

4-Allyl-2,6-dimethoxyphenol [3655; 6627-88-9], 1-Pentanol [2056; 71-41-0], 2-Amyl-3-phenyl-2-propen-1-ol [2065; 101-85-9], 4-Methoxybenzyl alkohol [2099; 105-13-5], Benzyl alkohol [2137; 100-51-6], Borneol [2157; 464-43-7, 507-70-0], 1-Butanol [2178; 71-36-3], Campholen alkohol [3741; 1901-38-8], Carvacrol [2245; 499-75-2], Carveol [2247; 2102-59-2, 99-48-9], Carvomenthol [3562; 499-69-4], Chavicol [4075; 501-92-8], Cinnamyl alkohol [2294; 104-54-1], Citronellol [2309; 106-22-9], I-alpha-Citronellol [2980; 6812-78-8], o-Cresol [3480; 95-48-7], m-Cresol [3530; 108-39-4], p-Cresol [2337; 106-44-5], Cymenol [3242; 1197-01-9]; Decadienol [3911; 18409-21-7]; 1-Decanol [2365; 112-30-1], 3-Decanol [3605; 1565-81-7]; 1-Decen-3-ol [3824; 51100-54-0]; (E)-2-Decen-1-ol [4304; 22104-80-9]; (Z)-4-Decen-1-ol [4349; 57074-37-0], Dehydrodihydroionol [3446; 57069-86-0], Dihydrocarveol [2379; 619-01-2], Dihydroeugenol [3598; 2785-87-7], Dihydrofarnesol [4031; 51411-24-6], Dihydroionol [3627; 3293-47-8], Syringol [3137; 91-10-1], Dimethylbenzyl alkohol [3139; 536-50-5], 4-(1,1-Dimethylethyl)phenol [3918; 98-54-4], 2,6-Dimethyl-4-heptanol [3140; 108-82-7], 2,6-Dimethyl-6-hepten-1-ol [3663; 36806-46-9], Dimethylnonadienol [4102; 67845-50-5], 3,7-Dimethyl-1-octanol [2391; 203-374-5], Hotrienol [3830; 20053-88-7], 2-Hydroxy-2-methyl-1-phenylpropan [2393; 100-86-7]; 2-Hydroxypiperiton [4143; 490-03-9], 1-Dodecanol [2617; 112-53-8], 2-(Ethoxymethyl)phenol [3485; 20920-83-6], Ethanol [2419; 64-17-5], Ethyl maltol [3487; 4940-11-8], 2-(3-Ethoxy-4-hydroxyphenyl)-4-methyl-1,3-dioxolane [3838; 68527-76-4], 4-Ethylsyringol [3671; 14059-92-8], Ethylguaiacol [2436; 2785-89-9]; 2-Ethyl-1-hexanol [3151; 104-76-7], 3-Ethyl-2-hydroxy-2-cyclopenten-1-one [3152; 21835-01-8]; 4-Ethylphenol [3156; 123-07-9], 3-Ethylthiobutan-1-ol [4282; 117013-33-9], 2-Ethylfenchol [3491; 18368-91-7], Eugenol [2467; 97-53-0], Farnesol [2478; 4602-84-0], Fenchol [2480; 1632-73-1], Furfurylalkohol [2491; 98-00-0], Geraniol [2507; 106-24-1], Glycerin [2525; 56-81-5], Glycerylmonooleat [2526; 25496-72-4], Guaiacol [2532; 90-05-1], (E,E)-Hepta-2,4-dien-1-ol [4127; 33467-79-7], 2-Heptanol [3288; 543-49-7], 3-Heptanol [3547; 589-82-2], (Z)-4-Hepten-1-ol [3841; 6191-71-5], 1-Hepten-3-ol [4129; 4938-52-7], 1-Heptanol [2548; 111-70-6], 1-Hexadecanol [2554; 36653-82-4], (E,E)-Hexa-2,4-dien-1-ol [3922; 111-28-4], 1-Hexanol [2567; 111-27-3], 3-Hexanol [3351; 623-37-0], 1-Hexen-3-ol [3608; 4798-44-1], (E)-2-Hexen-1-ol [2562; 2305-21-7], (Z)-2-Hexenol [3924; 928-94-9], (Z)-3-Hexen-1-ol [2563; 928-96-1], (E)-3-Hexen-1-ol [4356; 928-97-2]; 5-Hexen-1-ol [4351; 821-41-0], (E)-4-Hexen-1-ol [3430; 928-92-7], (Z)-4-Hexen-1-ol [3430; 928-91-6]; 4-Hydroxybenzyl alkohol [3987; 623-05-2], Hydroxycitronellol [2586; 107-74-4], alpha-Ionol [3624; 25312-34-9], beta-Ionol [3625; 22029-76-1]; Isoamylalkohol [2057; 123-51-3]; Isoborneol [2158; 124-76-5]; Isobutylalkohol [2179; 78-83-1]; 4-Methyl-1-phenyl-2-pentanol [2208; 7779-78-4]; Isochavicol [4062; 539-12-8], Isoeugenol [2468; 97-54-1], 2-Propanol [2929; 67-63-0]; Cuminol [2933; 536-60-7], o-Cumenol [3461; 88-69-7], Isopulegol [2962; 89-79-2], Linalooloxid [3746; 1365-19-1, 5989-33-3], Linalool [2635; 78-70-6], Maltol [2656; 118-71-8], Menthol [2665; 15356-60-2, 1490-04-6, 2216-51-5], 2-(L-Menthoxy)ethanol [4154; 38618-23-4], Menthyl lactate [3748; 59259-38-0], p-Menthane-3,8-diol [4053; 42822-86-6], (-)-3-Menthoxypropane-1,2-diol [3784; 87061-04-9], Mercaptobutanol [3502; 37887-04-0], 3-Mercaptohexanol [3850; 51755-83-0], 3-Mercapto-2-methyl-1-butanol [3993; 227456-33-9]; 3-Mercapto-3-methyl-1-butanol [3854; 34300-94-2]; 2-Mercapto-2-methyl-1-pentanol [3995; 258823-39-1], 3-Mercapto-2-methyl-1-pentanol [3996; 227456-27-1], 4-Mercapto-4-methyl-2-pentanol [4158; 31539-84-1], Creosol [2671; 93-51-6]; 4-Vinylguaiacol [2675; 7786-61-0], 2-Methylbutan-1-ol [3998; 137-32-6], 3-Methyl-2-butanol [3703; 598-75-4], 2-Methylbut-2-en-1-ol [4178; 4675-87-0], Prenol [3647; 556-82-1], 4-Methyl-2,6-dimethoxyphenol [3704; 6638-05-7], 3-Methyl-1-pentanol [3762; 589-35-5]; 2-Phenyl-1-propanol [2732; 1123-85-9], 2-Methyl-4-phenyl-2-butanol [3629; 103-05-9], 4-(Methylthio)-butanol [3600; 20582-85-8], 2-(Methylthio)ethanol [4004; 5271-38-5]; 3-Methylthio-1-hexanol [3438; 51755-66-9], Thioguaiacol [3210; 1073-29-6], 3-(Methylthio)-propanol [3415; 505-10-2], Myrtenol [3439; 515-00-4], Neomenthol [2666; 2216-52-6], Nerol [2770; 106-25-2], Nerolidol [2772; 142-50-7, 7212-44-4, 40716-66-3], (3E,6Z)-Nonadien-1-ol [3884; 53046-97-2]; (3Z,6Z)-Nonadien-1-ol [3885; 76649-25-7], 2,6-Nonadien-1-ol [2780; 7786-44-9], 2,4-Nonadien-1-ol [3951; 62488-56-6], (2E,6Z)-Nonadien-1-ol [2780; 28069-72-9], Nonan-2-ol [3315; 628-99-9], Nonan-1-ol [2789; 143-08-8], (Z)-2-Nonen-1-ol [3720; 41453-56-9], (Z)-6-Nonen-1-ol [3465; 35854-86-5], (E)-2-Nonen-1-ol [3379; 31502-14-4], Nopol [3938; 128-50-7], (E,E)-2,4-Octadien-1-ol [3956; 18409-20-6], Octan-1-ol [2800; 111-87-5], Octan-2-ol [2801; 123-96-6], Octan-3-ol [3581; 589-98-0], (E)-2-Octen-1-ol [3887; 18409-17-1], (E)-2-Octen-4-ol [3888; 4798-61-2], 1-Octen-3-ol [2805; 3391-86-4], 3-Octen-2-ol [3602; 76649-14-4], (Z)-3-Octen-1-ol [3467; 20125-84-2], (Z)-5-Octen-1-ol [3722; 64275-73-6], (Z)-4-Octen-1-ol [4354; 54393-36-1], Oleyl alkohol [4363; 143-28-2], 2-Pentanol [3316; 6032-29-7], 1-Penten-3-ol [3584; 616-25-1], cis-2-Pentenol [4305; 1576-95-0], Perilla alkohol [2664; 536-59-4], 2-Phenethyl alkohol [2858; 60-12-8], Phenol [3223; 108-95-2], 4-Phenyl-2-butanol [2879; 2344-70-9], 4-Phenyl-3-buten-2-ol [2880; 17488-65-2], 1-Phenyl-3-methyl-3-pentanol [2883; 10415-87-9], 5-Phenylpentanol [3618; 10521-91-2], 2-Phenylphenol [3959; 90-43-7], 1-Phenyl-1-propanol [2884; 93-54-9], 3-Phenyl-1-propanol [2885; 122-97-4], Phytol [4196; 150-86-7], Pinocarveol [3587; 5947-36-4], p-Menth-1-en-3-ol [3179; 491-04-3], 1-Propanol [2928; 71-23-8], 4-Propenyl-2,6-dimethoxyphenol [3728; 20675-95-0], 2-Ethoxy-5-(1-propenyl)phenol [2922; 94-86-0], Propylenglycol mono- und diester von Fettsäuren [4208], Propylenglycol stearat [2942; 142-75-6], Propylen glycol [2940; 57-55-6], 1-Phenyl-2-pentanol [2953; 705-73-7], 2-Propylphenol [3522; 644-35-9], 4-Propylphenol [3649; 645-56-7], 4-Propyl-2,6-dimethoxyphenol [3729; 6766-82-1], 3-Hydroxyphenol [3589; 108-46-3], Rhodinol [2980; 6812-78-8], 4-Thujanol [3239; 546-79-2], Santalol [3006; 11031-45-1, 115-71-9, 77-42-9], Sorbitol [3029; 50-70-4; E420], Sotolon [3634; 28664-35-9], Styrallyl alkohol [2685; 98-85-1], 4-Methyl-5-thiazoleethanol [3204; 137-00-8], p-Menth-3-en-1-ol [3563; 586-82-3], 1-p-Menthen-4-ol [2248; 562-74-3], p-Menth-8-en-1-ol [3564; 138-87-4], p-Menth-1-en-8-ol [3045, 98-55-5], Tetrahydrofurfuryl alkohol [3056; 97-99-4], 3,7-Dimethyloctan-3-ol [3060; 78-69-3], 1-Isopropyl-4-methylbicyclo[3.1.0]hexan-3-ol [4079; 21653-20-3], Thymol [3066; 89-83-8], 3,3,5-Trimethylcyclohexan-1-ol [3962; 116-02-9], 3,5,5-Trimethyl-1-hexanol [3324; 3452-97-9], 2,4,8-Trimethylnona-3,7-dien-2-ol [4211; 479547-57-4], 2,4,8-Trimethyl-7-nonen-2-ol [4212; 437770-28-0], 2,3,4-Trimethyl-3-pentanol [3903; 3054-92-0], 2,3,6-Trimethylphenol [3963; 2416-94-6], 2,4,6-Trimethylphenol [4329; 527-60-6], 2-Undecanol [3246; 1653-30-1], trans-Undec-2-en-1-ol [4068; 37617-03-1], 1-Undecanol [3097; 112-42-5], Vanillin butylenglycol acetal [4023; 4359-31-3], Vanillin menthoxypropanediol acetal [3904; 180964-47-0], Vanillin propylenglycol acetal [3905; 68527-74-2], Vanillyl alkohol [3737; 498-00-0], 2-Pinen-4-ol [3594; 473-67-6], Vetiverol [4217; 89-88-3], 4-Vinylphenol [3739; 2628-17-3], 2,5-Xylenol [3595; 95-87-4], 2,6-Xylenol [3249; 576-26-1], 3,4-Xylenol [3596; 95-65-8], 2',4',6'-Trihydroxy-3-(p-hydroxyphenyl)propiophenon [4390; 60-82-2], (+/-)-Ethyl-3-hydroxy-2-methylbutyrat [4391; 27372-03-8], 2,4-Dimethyl-4-nonanol [4407; 74356-31-3], 8,9-p-Menthen-1,2-diol [4409; 1946-00-5], Decahydro-2,2,4,8-tetramethyl-4,8-methanoazulen-9-ol [4410; 56747-96-7], D-2,8-p-Menthadien-1-ol [4411; 22771-44-4], (Z)-3-Nonen-1-ol [4412; 10340-23-5], 3,4-Dihydroxybenzoesäure [4430; 99-50-3], 3-Hydroxybenzoesäure [4431; 99-06-9], 2-Hydroxy-4-methoxybenzaldehyd [4435; 673-22-3], Methyl 3-hydroxybutyrat [4450; 1487-49-6], Ethyl 3-hydroxyoctanoat [4453; 7367-90-0], Hydroxyaceton [4462; 116-09-6], 1-Hydroxy-4-methyl-2-pentanon [4463; 68113-55-3], Propylenglycol mono-2-methylbutyrat [4467; 923593-56-0 & 923593-57-1], Propylenglycol monohexanoat [4469; 39556-41-7 & 170678-49-6], Dodecyl lactat [4482; 6283-92-7], Hexadecyl lactat [4483; 35274-05-6], Hydroxycitronellal propylenglycol acetal [4485; 93804-64-9], Citral glyceryl acetal [4486; 5694-82-6], Propylenglycol monobutyrat [4488; 29592-95-8], 2-Methoxy-6-(2-propenyl)phenol [4490; 579-60-2], (R)-(-)-1-Octen-3-ol [4492; 3687-48-7], Cubebol [4497; 23445-02-5], (-)-Sclareol [4502; 515-03-7], (+)-Cedrol [4503; 77-53-2], (D)-Limonen-10-ol [4504; 38142-45-9], p-Menthan-7-ol [4507; 5502-75-0], p-Menth-1-en-9-ol [4508; 18479-68-0], 2,2,6,7-Tetramethylbicyclo[4.3.0]nona-4,9(1)-dien-8-ol [4521; 97866-86-9], 6-Hydroxycarvon [4523; 51200-86-3], 2,6,6-Trimethyl-2-hydroxycyclohexanon [4531; 7500-42-7], Acetoin propylenglycol acetal [4532; 94089-23-3], (+/-)- N-Lactoyl tyramin [4550; 781674-18-8], Magnolol [4559; 528-43-8], Ethyl 2-hydroxyethyl sulfid [4562; 110-77-0], 2-Hydroxyethanethiol [4582; 60-24-2], Linalool oxid pyranoid [4593; 14049-11-7], 2-Hydroxy-5-methylacetophenon [4594; 1450-72-2], Ethyl 2-hydroxy-3-phenylpropionat [4598], (D)-Trehalose [4600; 6138-23-4], (E)-3-Nonen-1-ol [4605; 10339-61-4], Ethyl-5-hydroxyoctanoat [4610; 75587-05-2], (Z)-2-Octen-1-ol [4615; 26001-58-1], (E)-2-Tridecen-1-ol [4617; 68480-25-1], 2-(Phenoxy)ethanol [4620; 122-99-6], (4-Methylphenyl)methanol [4624; 589-18-4], Thiophen-2-ylmethanol [4642; 636-72-6], 2-Hydroxy-2-(4-hydroxy-3-methoxyphenyl)essigsäure [4660; 55-10-7], (4S)-4-Hydroxy-4-[(E,3R)-3-hydroxybut-1-enyl]-3,5,5-trimethylcyclohex-2-en-1-on [4661; 23526-45-6], 1-(4-Hydroxy-3-methoxyphenyl)decan-3-on [4665; 27113-22-0], (2R)-6-methyl-2-[(1R)-4-methyl-1-cyclohex-3-enyl]hept-5-en-2-ol [4666; 515-69-5], Mannitol [E421]; Isomalt [E953]; Maltitol [E965]; Lactitol [E966]; Xylitol [E967]; Erythritol [E968]; Fructose [57-48-7]; Glycose [50-99-7]; Sucrose [57-50-1]; Ethandiol [107-21-1].

Erfindungsgemäß besonders bevorzugte Verbindungen der Formel (I) sind auch solche, die durch Veresterung von teilweise oder vollständig hydrolysierten Speisefetten oder Ölen mit Lactisol herstellbar sind. Weiter bevorzugt sind Verbindungen der Formel (I) ausgewählt aus der Gruppe bestehend aus Verbindungen der Formel

**R1O-CH₂-CH(OR2)-CH₂-OR3** **(II)**

wobei mindestens einer der Reste R1, R2 oder R3 eine Lactisol-Gruppe der Formel (I) darstellt und die gegebenenfalls weiteren Reste jeweils unabhängig voneinander für Wasserstoff oder einen Fettsäurerest stehen.

Besonders bevorzugte Fettsäurereste sind dabei ausgewählt aus der Gruppe der Reste folgender Fettsäuren: Caprylsäure, Palmitinsäure, Stearinsäure, Myristinsäure, Ölsäure, Linolsäure, alpha-Linolensäure, gamma-Linolensäure, Palmitoleinsäure, Margarinsäure, Archinsäure, Lignocerinsäure, Elaidinsäure, Icosensäure, Cetoleinsäure, Behensäure, Stearidonsäure, Eicosapentaensäure, Docosahexaensäure, sowie Arachidonsäure.

Weitere erfindungsgemäß besonders bevorzugte Verbindungen der Formel (I) sind in den Beispielen 1 bis 6 weiter unten beschrieben.

Die im Rahmen der vorliegenden Erfindung beschriebenen erfindungsgemäßen Lactisolverbindungen weisen zahlreiche Vorteile auf. So wurde im Rahmen eigener Untersuchungen gefunden, dass insbesondere die erfindungsgemäßen (Prekursor-)Verbindungen der Formel (I) vorteilhafterweise weitgehend geschmacklos sind und/oder Geschmacksqualitäten anderer Substanzen, insbesondere eine süße Geschmacksqualität anderer Substanzen, nicht signifikant beeinflussen (vergleiche hierzu Anwendungsbeispiel 1 unten).

Zudem sind die erfindungsgemäßen Lactisolverbindungen dazu geeignet, (nach oraler Aufnahme) die Mundhöhle sowie den Magen zu passieren ohne dabei abgebaut bzw. gespalten zu werden. Im Darm setzen die erfindungsgemäßen Lactisolverbindungen dann einen oder mehrere unterschiedliche Süßrezeptor-Antagonisten frei, vorzugsweise einen oder mehrere unterschiedliche T1R2/T1R3-Rezeptor-Antagonisten (vergleiche hierzu Anwendungsbeispiel 2 unten). Die Verbindungen der Formel (I) sind vorteilhafterweise dazu geeignet, (nahezu ausschließlich) im Darm, insbesondere im Dünndarm, Lactisol freizusetzen. Diese Freisetzung wird nach bisherigen Erkenntnissen vor allem durch die im Milieu des Dünndarms aktiven Lipasen ermöglicht, welche insbesondere die Verbindungen der Formel (I) (wie oben beschrieben) in Lactisol und weitere Bestandteile (insbesondere Alkohole) spalten. Durch diese lokale Metabolisierung im Darm wird vorteilhafterweise ermöglicht, dass Lactisol selektiv die Süßrezeptoren in den Darmepithelzellen inhibiert, ohne (bei oraler Aufnahme) die Süßrezeptoren im Mundraum signifikant zu beeinflussen.

Ein besonderer Vorteil der erfindungsgemäßen Lactisolverbindungen besteht daher darin, dass diese
- direkt oral, insbesondere über die Nahrung, aufgenommen werden können, ohne den Geschmackseindruck im Mund signifikant zu beeinflussen, insbesondere ohne einen süßen Geschmackseindruck im Mundraum (signifikant) zu reduzieren, und dennoch
- dazu geeignet sind, den bzw. die entsprechenden Süßrezeptor-Antagonisten gezielt im Darm freizusetzen, so dass diese ihre medizinisch vorteilhafte Wirkung zur Prävention oder Behandlung von Krankheiten, insbesondere zur Prävention oder Behandlung von Diabetes (Typ 2), gezielt entfalten können (z.B. Lactisol als Süßrezeptor-Antagonist für T1R2/T1R3-Rezeptoren).

Die erfindungsgemäßen Lactisolverbindungen weisen gegenüber bisherigen Substanzen bzw. Methoden zur Beeinflussung der Süßrezeptoren im Darm (wie z.B. weiter oben im Zusammenhang mit WO 2009/026389 A2 beschrieben) zudem den Vorteil auf, dass sie unmittelbar zur Verarbeitung in Lebensmitteln geeignet sind und sich somit besser lebensmitteltechnisch verarbeiten lassen als Formulierungen, die neben einer entsprechenden freien Wirkstoff-Verbindung (gegebenenfalls unerwünschte) Hilfs- und/oder Zusatzstoffe beinhalten, um eine vorzeitige Wirkung zu verhindern. Insbesondere ist eine Umhüllung der Lactisolverbindungen zum Vermeiden einer Süßrezeptor-Inhibierung im Mundraum (durch freie Süßrezeptor-Antagonisten) und/oder zum Schutz vor einer vorzeitigen Metabolisierung (der Süßrezeptor-Antagonisten) in der Regel nicht nötig. Für die Zwecke der vorliegenden Erfindung wird die körpereigene Verdauung im Bereich des (Dünn-)Darms vielmehr gezielt zur selektiven Freisetzung der Rezeptor-Antagonisten aus den Lactisolverbindungen genutzt.

In eigenen Untersuchungen wurden exemplarisch die durch Veresterung von Lactisol mit den Alkoholen n-Hexanol, Glycerin, Ethandiol und n-Butanol hergestellten erfindungsgemäßen Lactisolverbindungen einer künstlichen Verdauung unterzogen (siehe Anwendungsbeispiel 2 unten). Dabei konnte beobachtet werden, dass Lactisol vorteilhafterweise (nahezu) ausschließlich im Darm freigesetzt wurde.

Zudem zeigen die erfindungsgemäßen Lactisolverbindungen, speziell die Verbindungen der Formeln (1) bis (4) (siehe die nachfolgenden Formelbilder sowie Anwendungsbeispiel 3 unten), vorteilhafterweise weder eine signifikante Aktivierung noch eine signifikante Inhibierung der T1R2/T1R3-Rezeptoren.

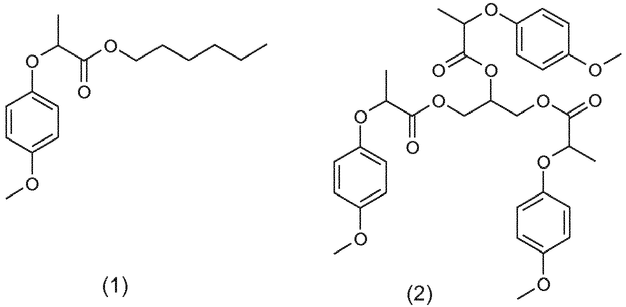

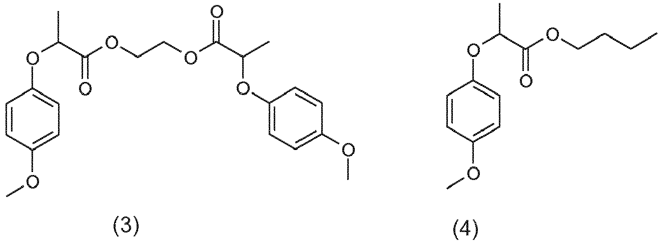

Die Verbindungen der Formeln (1) bis (4) sind im Rahmen der vorliegenden Erfindung besonders bevorzugte erfindungsgemäße Lactisolverbindungen.

Demnach betrifft die vorliegende Erfindung auch Lactisolverbindungen, die ausgewählt sind aus der Gruppe bestehend aus
**(1)** 2-(4-Methoxyphenoxy)-propansäurehexylester (Verbindung der Formel (1)),
**(2)** 2-(4-Methoxyphenoxy)propansäure-2,3-bis-[2-(4-methoxyphenoxy)-propionyloxy]-propylester (Verbindung der Formel (2)),
**(3)** 2-(4-Methoxyphenoxy)propansäure-2-[2-(4-methoxyphenoxy)-propionyloxy]-ethylester (Verbindung der Formel (3)),
**(4)** 2-(4-Methoxyphenoxy)propansäurebutylester (Verbindung der Formel (4)),
**(5)** 2-(4-Methoxyphenoxy)propansäure-(E)-hex-2-enylester und
**(6)** 2-(4-Methoxyphenoxy)-propansäure-2-isopropyl-5-methyl-phenylester, vorzugsweise aus der Gruppe bestehend aus den Verbindungen der Formeln (1) bis (4) 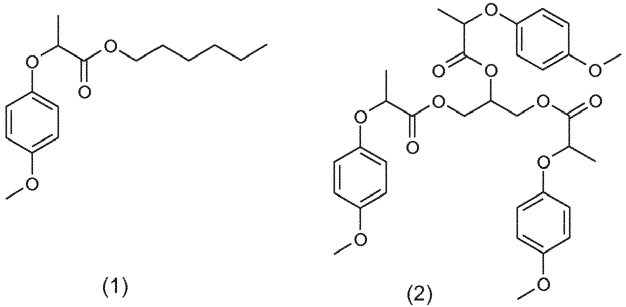 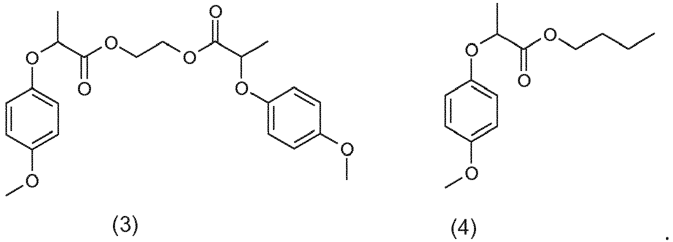 sowie deren Gemischen.

Besonders vorteilhaft für die Zwecke der vorliegenden Erfindung ist die Verbindung der Formel (2), so dass eine Lactisolverbindung oder eine Mischung aus zwei oder mehr Lactisolverbindungen, wobei eine der Substanzen eine Verbindung der Formel (2) ist.

Wie oben beschrieben, sind besonders bevorzugte Verbindungen der Formel (I) auch solche, die durch Veresterung von teilweise oder vollständig hydrolysierten Speisefetten oder Ölen mit Lactisol herstellbar sind.

### ARZNEIMITTEL UND PHARMAZEUTISCHE ANWENDUNGEN

Die erfindungsgemäßen Lactisolverbindungen eignen sich nicht nur zur Prävention oder Behandlung von Diabetes, insbesondere von Diabetes Typ 2, sondern auch zur
- zur Verminderung der Resorption von Glucose im Darm, insbesondere im Dünndarm, und/oder
- zur kompetitiven Hemmung von Süßrezeptoren im Darm, insbesondere zur kompetitiven Hemmung der T1R2/T1R3-Rezeptoren im Dünndarm.

Offenbart, jedoch nicht beansprucht, wird daher auch ein Verfahren zur
(a) Behandlung oder Prävention von Krankheiten, vorzugsweise zur Behandlung oder Prävention von Diabetes, insbesondere von Diabetes Typ 2,
(b) Verminderung der Resorption von Glucose im Darm, insbesondere im Dünndarm, und/oder
(c) kompetitiven Hemmung von Süßrezeptoren im Darm, insbesondere zur kompetitiven Hemmung der T1R2/T1R3-Rezeptoren im Dünndarm,
in Menschen oder Tieren, mit folgendem Schritt:
Verabreichen einer wirksamen Menge
   - einer Lactisolverbindung oder
   - einer Mischung aus zwei oder mehr Lactisolverbindungen der Formel (I), insbesondere einer solchen wie vorangehend als besonders bevorzugt beschrieben.

Ein solches Verfahren ist geeignet zur
(a) Behandlung oder Prävention von Krankheiten, vorzugsweise zur Behandlung oder Prävention von Diabetes, insbesondere von Diabetes Typ 2,
(b) Verminderung der Resorption von Glucose im Darm, insbesondere im Dünndarm, und/oder
(c) kompetitiven Hemmung von Süßrezeptoren im Darm, insbesondere zur kompetitiven Hemmung der T1R2/T1R3-Rezeptoren im Dünndarm,
in Menschen oder Tieren, die einer Behandlung mit Süßrezeptor-Antagonisten und/oder einer Diabetes-Behandlung bzw. -Prophylaxe bedürfen.

Die Lactisolverbindungen werden dabei vorzugsweise in Form einer erfindungsgemäßen Zubereitung, insbesondere in Form einer erfindungsgemäßen pharmazeutischen Zubereitung zur oralen Applikation (wie oben beschrieben), verabreicht.

### Ebenfalls zum Offenbarungsgehalt der vorliegenden Erfindung gehört

- eine erfindungsgemäße Lactisolverbindung oder
- eine erfindungsgemäße Mischung aus zwei oder mehr Lactisolverbindungen, wie jeweils oben beschrieben, vorzugsweise wie oben als bevorzugt beschrieben, oder
- eine erfindungsgemäße zum Verzehr geeignete Zusammensetzung (wie oben beschrieben) oder
- eine erfindungsgemäße der Ernährung oder dem Genuss dienende oder eine pharmazeutische Zubereitung (wie oben beschrieben)
zur Anwendung in einem Verfahren zur
(a) Behandlung oder Prävention von Krankheiten, vorzugsweise zur Behandlung oder Prävention von Diabetes, insbesondere von Diabetes Typ 2,
(b) Verminderung der Resorption von Glucose im Darm, insbesondere im Dünndarm, und/oder
(c) kompetitiven Hemmung von Süßrezeptoren im Darm, insbesondere zur kompetitiven Hemmung der T1R2/T1R3-Rezeptoren im Dünndarm,
in Menschen oder Tieren, die einer Behandlung mit Süßrezeptor-Antagonisten bedürfen.

Die erfindungsgemäßen Lactisolverbindungen oder deren Mischungen können Bestandteil einer der Ernährung oder dem Genuss dienenden Zubereitung oder einer pharmazeutischen Zubereitung sein sowie für die Herstellung einer pharmazeutischen Zubereitung (insbesondere eines Arzneimittels) zur Prävention oder Behandlung von Diabetes, insbesondere von Diabetes Typ 2 verwendet werden.

Besonders bevorzugt ist eine erfindungsgemäße Verwendung (wie oben beschrieben), wobei die pharmazeutische Zubereitung (das Arzneimittel) eine Zubereitung zur oralen Applikation ist.

Zudem bevorzugt ist eine Verwendung (wie oben beschrieben), wobei die pharmazeutische Zubereitung (das Arzneimittel) in einer Form ausgebildet ist, die ausgewählt ist aus der Gruppe bestehend aus: Kapseln, Tabletten, Dragees, Granulaten, Pellets, Feststoffmischungen, Dispersionen in flüssigen Phasen, Emulsionen, Pulver, Lösungen, Pasten.

### NAHRUNGSMITTEL

Im Folgenden werden erfindungsgemäße zum Verzehr geeignete Zusammensetzungen, der Ernährung oder dem Genuss dienende Zubereitungen oder Halbfertigwaren sowie erfindungsgemäße pharmazeutische Zubereitungen beschrieben.

Demnach betrifft die vorliegende Erfindung auch eine zum Verzehr geeignete Zusammensetzung, der Ernährung oder dem Genuss dienende Zubereitung oder Halbfertigware, bestehend aus
(a) einer Lactisolverbindung der Formel (I) sowie
(b) einem oder mehreren zum Verzehr geeigneten weiteren Bestandteilen.

Erfindungsgemäß bevorzugt ist eine zum Verzehr geeignete Zusammensetzung (wie oben beschrieben), wobei der bzw. einer, mehrere oder sämtliche der zum Verzehr geeigneten weiteren Bestandteile (b) ausgewählt ist bzw. sind aus der Gruppe bestehend aus
- süß schmeckenden Stoffen,
- süß riechenden Aromastoffen und
- Stoffen, vorzugsweise Maltodextrinen oder Stärke, die bei der Verdauung Glucose freisetzen.

### Süß schmeckende Stoffe

Besonders bevorzugt werden die erfindungsgemäßen Lactisolverbindungen demnach in Kombination mit süß schmeckenden Stoffen und/oder Aromastoffen eingesetzt, die einen süßen Geruch erzeugen oder vermitteln können, und/oder (vorzugsweise) in Kombination mit Stoffen, vorzugsweise Maltodextrinen oder Stärke, die bei der Verdauung (insbesondere im Mundraum) Glucose freisetzen.

Die süß schmeckenden Stoffe sind dabei vorzugsweise ausgewählt aus der Gruppe bestehend aus: süß schmeckende Kohlenhydrate (z.B. Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd), Zuckeralkohole (z.B. Erythritol, Threitol, Arabitol, Ribitol, Xylitol, Sorbitol, Mannitol, Dulcitol, Lactitol), Proteine (z.B. Miraculin, Pentaidin, Monellin, Thaumatin, Curculin, Brazzein), Süßstoffe wie z.B. Magap, Natriumcyclamat, Acesulfam K, Neohesperidindihydrochalkon, Naringindihydrochalkon, Saccharin, Saccharin-Natriumsalz, Aspartam, Superaspartam, Neotam, Alitam, Sucralose, Lugduname, Carrelame, Sucrononate, Sucrooctate, oder natürlich vorkommende Süßstoffen bestehend aus Miraculin, Curculin, Monellin, Mabinlin, Thaumatin, Curculin, Brazzein, Pentadin, D-Phenylalanin, D-Tryptophan, oder aus natürlichen Quellen gewonnenen Extrakte oder Fraktionen, enthaltend diese Aminosäuren und/oder Proteine, Neohesperidindihydrochalkon, Steviolgylcoside, Stevioside, Steviolbiosid, Rebaudioside, Rebaudiosid A, Rebaudiosid B, Rebaudiosid C, Rebaudiosid D, Rebaudiosid E, Rebaudiosid F, Rebaudiosid G, Rebaudiosid H, Dulcoside, Rubusoside, Suavioside A, Suavioside B, Suavioside G, Suavioside H, Suavioside I, Suavioside J, Baiyunoside 1 Baiyunoside 2, Phlomisoside 1, Phlomisoside 2, Phlomisoside 3, sowie Phlomisoside 4, Abrusoside A, Abrusoside B, Abrusoside C, Abrusoside D, Cyclocaryoside A und Cyclocaryoside I, Oslandin, Polypodosid A, Strogin 1, Strogin, 2 Strogin 4, Selligueanin A, Dihydroquercetin-3-acetat, Perillartin, Telosmosid A₁₅, Periandrin I-V, Pterocaryoside, Cyclocaryoside, Mukurozioside, trans-Anethol, trans-Cinnamaldehyd, Bryoside, Bryonoside, Bryonodulcoside, Carnosifloside, Scandenoside, Gypenoside, Trilobatin, Phloridzin, Dihydroflavanole, Hematoxylin, Cyanin, Chlorogensäure, Albiziasaponin, Telosmoside, Gaudichaudiosid, Mogroside, Hernandulcine, Monatin, Glycyrrhetinsäure und deren Derivate und Salze und Phyllodulcin, wobei im Falle der natürlich vorkommenden Süßstoffe auch Extrakte oder angereicherte Fraktionen dieser Extrakte verwendet werden können, z.B. Thaumatococcus-Extrakte (Katemfestaude), Extrakte aus *Stevia* ssp. (insbesondere *Stevia rebaudiana*), Swingle-Extrakt (*Momordica* bzw. *Siratia grosvenorii,* Luo-Han-Guo), Extrakte aus *Glycerrhyzia* ssp. (insb. *Glycerrhyzia glabra*), *Rubus* ssp. (insbesondere *Rubus suavissimus*), Citrus-Extrakte, Extrakte aus *Lippia dulcis,* Buddha-Tee-Extrakte (*Hydrangea dulcis* oder *Hydrangea macrophylla*).

### Süß riechende Aromastoffe

Erfindungsgemäß vorteilhaft mit den erfindungsgemäßen Lactisolverbindungen zu kombinierende Aromastoffe, die einen süßen Geruchseindruck erzeugen oder vermitteln können, sind vorzugsweise ausgewählt aus der Gruppe bestehend aus: Vanillin, Ethylvanillin, 2-Hydrox-4-methoxybenzaldehyd, Ethylvanillinisobutyrat (= 3-Ethoxy-4-isobutyryloxybenzaldehyd), Furaneol® (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al und Phenylacetaldehyd.

### Lebensmittel

Die erfindungsgemäßen zum Verzehr geeigneten Zusammensetzungen, der Ernährung oder dem Genuss dienenden Zubereitungen oder Halbfertigwaren sind regelmäßig Produkte, die dazu bestimmt sind, in die menschliche Mundhöhle eingebracht zu werden, dort eine bestimmte Zeit zu verbleiben und anschließend entweder verzehrt (z.B. verzehrfertige Lebensmittel, siehe hierzu weiter unten) oder wieder aus der Mundhöhle entfernt zu werden (z.B. Kaugummis). Zu diesen Produkten gehören dabei sämtliche Erzeugnisse oder Stoffe, die dazu bestimmt sind, in verarbeitetem, teilweise verarbeitetem oder unverarbeitetem Zustand vom Menschen aufgenommen zu werden. Insbesondere sind zum Verzehr geeignete Zusammensetzungen Erzeugnisse, die Lebensmitteln bei ihrer Herstellung, Verarbeitung oder Bearbeitung zugesetzt werden und dazu vorgesehen sind, in die menschliche Mundhöhle eingebracht zu werden, insbesondere mit dem besagten Lebensmittel. Demnach können solche Zusammensetzungen insbesondere auch in der Ernährung oder dem Genuss dienenden gebrauchs- oder verzehrfertigen Zubereitungen enthalten sein (der Ernährung oder dem Genuss dienende gebrauchs- oder verzehrfertige Zubereitungen sind im Rahmen des vorliegenden Textes insbesondere Lebensmittel, speziell verzehrfertige Lebensmittel (s. Definition weiter unten)). Zudem können solche Zusammensetzungen Bestandteil einer Halbfertigware sein, welche gegebenenfalls wiederum zur Herstellung von der Ernährung oder dem Genuss dienenden gebrauchs- oder verzehrfertigen Zubereitungen verwendet werden kann (erfindungsgemäße der Ernährung oder dem Genuss dienende Gebrauchs- oder verzehrfertige Zubereitungen und Halbfertigwaren werden weiter unten weiter beschrieben).

Im Rahmen des vorliegenden Textes werden unter einem "Lebensmittel" insbesondere Stoffe verstanden, die dazu bestimmt sind, in unverändertem, zubereitetem oder verarbeitetem Zustand vom Menschen geschluckt und dann verdaut zu werden: Als Lebensmittel werden insoweit auch Umhüllungen, Überzüge oder sonstige Umschließungen verstanden, die dazu bestimmt sind, mitverschluckt zu werden, oder bei denen ein Verschlucken vorauszusehen ist. Auch bestimmte Produkte, die üblicherweise wieder aus der Mundhöhle entfernt werden (z.B. Kaugummis) sind im Rahmen des vorliegenden Textes als Lebensmittel zu verstehen, da bei ihnen nicht auszuschließen ist, dass sie zumindest teilweise geschluckt werden.

Unter einem verzehrfertigen Lebensmittel ist im Rahmen des vorliegenden Textes ein Lebensmittel zu verstehen, das hinsichtlich der für den Geschmack maßgeblichen Substanzen bereits vollständig zusammengesetzt ist. Unter den Begriff "verzehrfertiges Lebensmittel" fallen auch Getränke sowie feste oder halbfeste verzehrfertige Lebensmittel. Als Beispiele seien genannt Tiefkühlprodukte, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln.

### HALBFERTIGWAREN

Unter einer Halbfertigware ist im Zusammenhang des vorliegenden Textes ein Produkt zu verstehen, welches aufgrund eines sehr hohen Gehaltes an Aroma- und/oder Geschmacksstoffen für die Verwendung als verzehrfertiges Lebensmittel (grundsätzlich) ungeeignet ist. Erst durch Mischen mit mindestens einem weiteren Bestandteil (d.h. durch Verringern der Konzentration der betreffenden Aroma- und/oder Geschmacksstoffe) und gegebenenfalls weitere Prozessschritte (z.B. Erwärmen, Gefrieren) wird die Halbfertigware in ein verzehrfertiges Lebensmittel überführt. Als Beispiel für Halbfertigwaren seien hier Tütensuppen, Backaromen und Puddingpulver genannt.

### Kaugummis

Kaugummis (als bevorzugte erfindungsgemäße Lebensmittel bzw. der Ernährung oder den Genuss dienende Zubereitung) umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkohole (insbesondere Sorbitol, Xylitol, Mannitol), Kühlwirkstoffe, Geschmackskorrigenzien für unangenehme Geschmackseindrücke, weitere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Stabilisatoren, Geruchskorrigentien und Aromen (z.B.: Eucalyptus-Menthol, Kirsche, Erdbeere, Grapefruit, Vanille, Banane, Citrus, Pfirsich, schwarze Johannisbeere, Tropenfrüchte, Ingwer, Kaffee, Zimt, Kombinationen (der genannten Aromen) mit Mintaromen sowie Spearmint und Pfefferminz alleine). Besonders interessant ist auch die Kombination dieser Aromen mit weiteren Stoffen, die kühlende, wärmende und/oder mundwässernde Eigenschaften aufweisen.

Im Stand der Technik sind zahlreiche unterschiedliche Kaugummibasen bekannt, wobei zwischen sogenannten "chewing gum"- und "bubble gum"-Basen zu unterscheiden ist, wobei letztere weicher sind, damit sich damit auch Kaugummiblasen bilden lassen. Gängige Kaugummibasen umfassen neben traditionell eingesetzten natürlichen Harzen oder dem Naturlatex Chicle heute zumeist Elastomere wie Polyvinylacetate (PVA), Polyethylene, (nieder- oder mittelmolekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispielsweise in EP 0 242 325, US 4,518,615, US 5,093,136, US 5,266,336 US 5,601,858 oder US 6,986,709 beschrieben. Daneben umfassen Kaugummibasen ggf. weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearinsäure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerinmonostearat

Im Folgenden werden erfindungsgemäß bevorzugte zum Verzehr geeignete Zusammensetzungen beschrieben. Hierbei gilt grundsätzlich für die Auswahl der Lactisolverbindung(en) bzw. die Verbindungen der Formel (I) das vorangehend Gesagte entsprechend. Die vorangehend als bevorzugt bezeichneten Lactisolverbindungen, insbesondere die Verbindungen der Formel (I), sind demnach besonders bevorzugt in Bestandteil (A) einer erfindungsgemäßen Zusammensetzung enthalten.

### Trägerstoffe

Erfindungsgemäße Zusammensetzungen (wie oben beschrieben) umfassen als einen oder mehrere zum Verzehr geeignete weitere Bestandteile vorzugsweise einen oder mehrere feste Trägerstoffe. Solche Zusammensetzungen sind zudem bevorzugt sprühgetrocknete Zusammensetzungen. Ein weiterer Gegenstand der vorliegenden Erfindung umfasst daher Zusammensetzungen bestehend aus
(a) einer Lactisolverbindung der Formel (I) und
(b) festen Trägerstoffen,
wobei die Gesamtmenge an Bestandteil (a) und festen Trägerstoffen (b) in der Zusammensetzung, bezogen auf die Trockenmasse der Zusammensetzung, 70 bis 100 Gew.-% beträgt.

In solchen erfindungsgemäßen (vorzugsweise sprühgetrockneten) Zusammensetzungen sind der eine bzw. mehrere oder sämtliche der enthaltenen Trägerstoffe vorzugsweise ausgewählt aus der Gruppe bestehend aus Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate, vorzugsweise Maltodextrine und Dextrine), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin und Xanthan Gum. Besonders bevorzugte Trägerstoffe sind Siliciumdioxid, Gummi Arabicum und Maltodextrine, wobei hier wiederum Maltodextrine mit DE-Werten (dextrose equivalent) im Bereich von 5 bis 20 bevorzugt sind. Es ist dabei unerheblich, welche Pflanze ursprünglich die Stärke zur Herstellung der Stärkehydrolysate geliefert hat. Geeignet und leicht verfügbar sind Mais-basierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

Die erfindungsgemäßen zum Verzehr geeigneten Zusammensetzungen werden vorzugsweise mittels Sprühtrocknung oder durch mechanische Mischvorgänge, wobei gleichzeitig auch eine Zerkleinerung der vorliegenden Partikel (insbesondere der oben genannten Trägerstoffe) erfolgen kann, hergestellt. Erfindungsgemäße Zusammensetzungen umfassend einen oder mehrere zum Verzehr geeignete feste Trägerstoffe (wie oben beschrieben), die mittels Sprühtrocknung hergestellt sind, sind besonders bevorzugt. Hinsichtlich des Verfahrens der Sprühtrocknung wird verwiesen auf US 3,159,585, US 3,971,852, US 4,532,145 und US 5,124,162.

Dabei sind erfindungsgemäße sprühgetrocknete Zusammensetzungen besonders bevorzugt, welche eine mittlere Partikelgröße im Bereich von 30 bis 300 µm und/oder eine Restfeuchte von 5 Gew.-% oder weniger, bezogen auf das Gesamtgewicht der Zusammensetzung, besitzen.

Besonders bevorzugt liegt in einer solchen Zusammensetzung das Gewichtsverhältnis der Gesamtmenge an Bestandteil (A) zur Gesamtmenge an festen Trägerstoffen in der Zusammensetzung, jeweils bezogen auf die Trockenmasse der Zusammensetzung, im Bereich von 1 : 10 bis 1 : 100000, bevorzugt im Bereich von 1 : 50 bis 1 : 20000, besonders bevorzugt im Bereich von 1 : 100 bis 1 : 5000.

Außerdem ist es bevorzugt, wenn die Gesamtmenge an Bestandteil (A) und festen Trägerstoffen in der erfindungsgemäßen Zusammensetzung (wie oben beschrieben), bezogen auf die Trockenmasse der Zusammensetzung, 70 bis 100 Gew.-%, vorzugsweise 85 bis 100 Gew.-%, beträgt.

Gemäß einem weiteren Aspekt der vorliegenden Erfindung umfasst eine erfindungsgemäße zum Verkehr geeignete Zusammensetzung als einen oder mehrere zum Verzehr geeignete weitere Bestandteile einen oder mehrere flüchtige Aromastoffe, d.h. einen einzelnen flüchtigen Aromastoff oder eine Mischung unterschiedlicher flüchtiger Aromastoffe bzw. eine Aromakomposition.

Im Rahmen des vorliegenden Textes sind erfindungsgemäße Lactisolverbindungen (wie oben beschrieben), insbesondere Verbindungen der Formel (I), nicht den weiteren Bestandteilen (B) zuzuordnen.

Vorzugsweise besitzen ein, mehrere oder sämtliche der in einer erfindungsgemäßen Zusammensetzung enthaltenen flüchtigen Aromastoffe einen Dampfdruck von größer oder gleich 0,01 Pa bei 25°C, vorzugsweise einen Dampfdruck von größer oder gleich 0,025 Pa bei 25°C. Weiter bevorzugt weist der bzw. weisen die flüchtigen Aromastoffe, zumindest aber ein Großteil der flüchtigen Aromastoffe, einen Dampfdruck von größer oder gleich 1 Pa bei 25°C auf.

Besonders bevorzugte (flüchtige) Aromastoffe finden sich z.B. in H. Surburg und J. Panten, Common Fragrance and Flavor Materials, 5th. Ed., Wiley-VCH, Weinheim 2006. Es seien beispielsweise genannt: organische Säuren (gesättigt und ungesättigt) wie z.B. Buttersäure, Essigsäure, Methylbuttersäure, Capronsäure; Alkohole (gesättigt und ungesättigt) wie z.B. Ethanol, Propylenglykol, Octenol, cis-3-Hexenol, Benzylalkohol; Sulfide und Disulfide wie z.B. Dimethylsulfid, Difurfuryldisulfid, Methylthiopropanal; Thiole wie z.B. Methylfuranthiol; Pyrazine und Pyrroline wie z.B. Methylpyrazin, Acetylpyrazin, 2-Propionylpyrrolin, 2-Acetylpyrrolin.

Erfindungsgemäß kann eine Aromakomposition als Bestandteil einer erfindungsgemäßen Zusammensetzung auch in Form von Reaktionsaromen (Maillard-Produkte) und/oder Extrakten bzw. ätherischen Ölen von Pflanzen oder Pflanzenteilen bzw. Fraktionen davon eingesetzt werden.

Eine erfindungsgemäße Zusammensetzung (wie oben beschrieben) umfassend einen oder mehrere flüchtige Aromastoffe bzw. eine Aromakomposition (sowie einen oder mehrere zum Verzehr geeignete Trägerstoffe) ist gemäß einer bevorzugten, alternativen Ausgestaltung der vorliegenden Erfindung selbst eine (erfindungsgemäße) Aromakomposition.

Gemäß einem weiteren Aspekt der vorliegenden Erfindung ist eine erfindungsgemäße Zusammensetzung (wie oben beschrieben) vorzugsweise eine Wasser-in-Öl (W/O)-Emulsion. Dabei gilt hinsichtlich der bevorzugt enthaltenen Lactisolverbindungen bzw. Mischungen das vorangehend Gesagte entsprechend.

Gemäß den vorangehend beschriebenen Aspekten der vorliegenden Erfindung ist eine zum Verzehr geeignete Zusammensetzung (wie oben beschrieben) besonders bevorzugt, wobei Bestandteil (b)
- einen oder mehrere feste Trägerstoffe sowie gegebenenfalls eine Aromakomposition (siehe oben) oder
- Wasser, eine Ölphase, einen oder mehrere W/O-Emulgatoren, gegebenenfalls ein oder mehrere Antioxidantien sowie gegebenenfalls einen oder mehrere Stoffe zur Verstärkung einer antioxidativen Wirkung
umfasst oder daraus besteht.

Die festen Trägerstoffe sowie die Aromakomposition können dabei einen oder mehrere vorangehend beschriebene süß schmeckende Stoffe, süß riechende Aromastoffe und/oder Stoffe, vorzugsweise Maltodextrine oder Stärke, die bei der Verdauung Glucose freisetzen, umfassen oder daraus bestehen. Insbesondere kann der bzw. können die festen Trägerstoffe aus Stoffen ausgewählt sein, die bei der Verdauung Glucose freisetzen.

Dabei ist eine erfindungsgemäße Zusammensetzung (wie oben beschrieben) besonders bevorzugt, wobei Bestandteil (b) einen oder mehrere feste Trägerstoffe sowie gegebenenfalls eine Aromakomposition umfasst oder daraus besteht und das Gewichtsverhältnis der Gesamtmenge an Bestandteil (A) in der Zusammensetzung zur Gesamtmenge an festen Trägerstoffen in der Zusammensetzung, jeweils bezogen auf die Trockenmasse der Zusammensetzung, im Bereich von 1:10 bis 1:100000, vorzugsweise im Bereich von 1:50 bis 1:20000, besonders bevorzugt im Bereich von 1:100 bis 1:5000, liegt.

Zudem bevorzugt ist eine zum Verzehr geeignete Zusammensetzung (wie oben beschrieben), wobei die Gesamtmenge an Bestandteil (a) und festen Trägerstoffen in der Zusammensetzung, bezogen auf die Trockenmasse der Zusammensetzung vorzugsweise 85 bis 100 Gew.-%, beträgt.

### Emulsionen

Ferner bevorzugt ist eine zum Verzehr geeignete Zusammensetzung (wie oben beschrieben), wobei die Gesamtmenge an Bestandteil (a) bezogen auf das Gesamtgewicht der Zusammensetzung 0,01 bis 0,1 Gew.-% beträgt und Bestandteil (b)
- jeweils bezogen auf das Gesamtgewicht der Zusammensetzung
   5 bis 30 Gew.-%, vorzugsweise 8 bis 25 Gew.-% Wasser,
   50 bis 90 Gew.-%, vorzugsweise 60 bis 80 Gew.-% Ölphase,
   0,1 bis 5 Gew.-% W/O-Emulgator/en und
- gegebenenfalls ein oder mehrere Antioxidantien sowie
- gegebenenfalls einen oder mehrere Stoffe zur Verstärkung einer anti-oxidativen Wirkung
umfasst oder (vorzugsweise) daraus besteht.

Die Ölphase einer solchen erfindungsgemäßen W/O-Emulsion umfasst vorzugsweise ein fettes Öl und/oder eine Aromakomposition. Bevorzugt sind Ölphasen umfassend oder bestehend aus einem fetten Öl und einer Aromakomposition, vorzugsweise einer Aromakomposition wie oben als bevorzugt beschrieben.

Als fette Öle eignen sich beispielsweise Speiseöle, insbesondere Pflanzenöle. Geeignete fette Öle sind beispielsweise Borretschöl, Distelöl, Erdnussöl, Haselnussöl, Kokosöl, Kürbiskernöl, Leinöl, Maiskeimöl, Makadamianussöl, Mandelöl, Olivenöl, Palmkernöl, Pekannussöl, Pistazienkernöl, Rapsöl, Reiskeimöl, Sesamöl, Sojaöl, Sonnenblumenöl, Walnussöl oder Weizenkeimöl, oder daraus erhältliche Fraktionen. Es können auch flüssige Neutralester basierend auf mittelkettigen Fettsäuren und Glycerin verwendet werden, wie beispielsweise Miglyole (z.B. Miglyol 810, Miglyol 812). Bevorzugt sind Sonnenblumenöl, Palmkernöl und Rapsöl. Ferner bevorzugt werden fraktionierte Kokosöle verwendet, die hauptsächlich Fettsäurereste mit 6 bis 8 Kohlenstoffatomen aufweisen. Diese zeichnen sich durch ihre Geschmacksneutralität sowie durch ihre gute Oxidationsstabilität aus.

Vorzugsweise wird der verzehrbare W/O-Emulgator ausgewählt aus der Gruppe bestehend aus Lecithin (E 322), Mono- und Diglyceride von Speisefettsäuren (E 471), Essigsäuremonoglyceride (E 472a), Milchsäuremonoglyceride (E 472b), Citronensäuremonoglyceride (E 472c), Weinsäuremonoglyceride (E 472d), Diacetylweinsäuremonoglyceride (E 472e) und Sorbitanmonostearat (E 491).

Erfindungsgemäß bevorzugt einzusetzende Antioxidantien und Stoffe, welche die antioxidative Wirkung verstärken können, sind vorzugsweise ausgewählt aus der Gruppe bestehend aus: natürlich vorkommenden Tocopherole und deren Derivate, Tocotrienole, Flavonoide, Ascorbinsäure und ihre Salze, alpha-Hydroxysäuren (z.B. Citronensäure, Milchsäure, Äpfelsäure, Weinsäure) und deren Na-, K- und Ca-Salze, aus Pflanzen isolierte Inhaltsstoffe, Extrakte bzw. Fraktionen davon z.B. aus Tee, Grüntee, Algen, Traubenkernen, Weizenkeimen, Rosmarin, Oregano, Flavonoide, Quercetin, phenolische Benzylamine. Weiterhin sind als Antioxidantien Propylgallat, Octylgallat, Dodecylgallat, Butylhydroxyanisol (BHA), Butylhydroxytoluol (BHT), Lecithine, Mono- und Diglyceride von Speisefettsäuren verestert mit Citronensäure, Orthophosphate und Na-, K- und Ca-Salze der Monophosphorsäure und Ascorbylpalmitat geeignet.

Die erfindungsgemäßen W/O-Emulsionen eignen sich vorteilhafterweise besonders gut zum Aufbringen auf die Oberfläche von Lebensmitteln, wobei die Lebensmittel vorzugsweise einen Wassergehalt von 10 Gew.-% oder weniger, besonders bevorzugt von 5 Gew.-% oder weniger, besitzen. In einer bevorzugten Ausführungsform weist die erfindungsgemäße W/O-Emulsion hierfür bei Aufbringungstemperatur eine ausreichend niedrige Viskosität auf, so dass ein Aufbringen der W/O-Emulsion mittels Sprühen möglich ist. Bevorzugte Lebensmittel, auf deren Oberflächen eine erfindungsgemäße W/O-Emulsion aufgebracht werden kann, sind beispielsweise Cracker, Chips (z.B. auf Basis von Kartoffeln, Mais, Getreide oder Brot), extrudierte Knabberartikel (Snacks, z.B. Flips) oder Laugengebäck (z.B. Salzstangen). Erfindungsgemäße W/O-Emulsionen werden vorzugsweise in einer Menge von 0,5 bis 6 Gew.-% auf die Lebensmitteloberflächen aufgebracht, bezogen auf das Gesamtgewicht des Lebensmittels.

Wie bereits erwähnt, betrifft die vorliegende Erfindung auch der Ernährung oder dem Genuss dienende Zubereitungen, insbesondere gebrauchs- oder verzehrfertige Zubereitungen und Halbfertigwaren (wie oben beschrieben).

Besonders bevorzugt ist eine der Ernährung oder dem Genuss dienende Zubereitung oder Halbfertigware (wie oben beschrieben), umfassend eine erfindungsgemäße zum Verzehr geeignete Zusammensetzung (wie oben beschrieben).

Vorzugsweise sind in einer erfindungsgemäßen Zusammensetzung oder Zubereitung, insbesondere in einer der Ernährung oder dem Genuss dienenden oder einer pharmazeutischen Zubereitung (wie oben beschrieben) die erfindungsgemäßen bzw. die erfindungsgemäß einzusetzenden Lactisolverbindungen, insbesondere die Verbindungen der Formel (I), in einer wirksamen Menge enthalten. D.h., die in einer erfindungsgemäßen Zusammensetzung oder insbesondere Zubereitung enthaltene Menge an Lactisolverbindungen ist vorzugsweise so hoch, dass die (nach oraler Aufnahme) im Darm freigesetzten Süßrezeptor-Antagonisten (z.B. Lactisol, siehe oben) ausreichen, um die Süßrezeptoren im Darm, insbesondere die T1R2/T1R3-Rezeptoren im Dünndarm, signifikant zu hemmen. Weiter bevorzugt ist es, wenn die im Darm freigesetzten Süßrezeptor-Antagonisten ausreichen, um die Resorption von Glucose im Darm, insbesondere im Dünndarm, zu vermindern.

Alternativ kann eine erfindungsgemäße Zubereitung auch eine Gesamtmenge an Lactisolverbindungen (wie oben beschrieben) umfassen, die an sich unterhalb der wirksamen Menge liegt, so dass erst (bei oraler Aufnahme) in Kombination mit weiteren erfindungsgemäßen Zubereitungen die Schwelle der wirksamen Menge erreicht oder übertroffen wird.

Erfindungsgemäße der Ernährung oder dem Genuss dienende Zubereitungen oder Halbfertigwaren sind vorzugsweise zucker-haltige, zucker-reduzierte oder gesüßte zuckerfreie Zubereitungen oder Halbfertigwaren.

Der Begriff "zucker-reduziert" bedeutet im Rahmen der vorliegenden Erfindung, dass die erfindungsgemäße Zubereitung bzw. Halbfertigware vorzugsweise deutlich weniger Zucker bzw. kalorische Süßungsmittel im Allgemeinen gegenüber der üblichen Zubereitung bzw. Halbfertigware enthält.

Besonders bevorzugt sind erfindungsgemäße der Ernährung oder dem Genuss dienende Zubereitungen oder Halbfertigwaren (wie oben beschrieben), wobei Bestandteil (B)
- einen oder mehrere süß schmeckende Stoffe und/oder
- einen oder mehrere süß riechende Aromastoffe (Aromastoffe, die einen süßen Geruchseindruck erzeugen oder vermitteln können)
umfassen oder daraus bestehen. Bevorzugte süß schmeckende Stoffe und süß riechende Aromastoffe bzw. Aromastoffe, die einen süßen Geruchseindruck erzeugen oder vermitteln können, sind weiter oben genannt.

Insbesondere erfindungsgemäße der Ernährung oder dem Genuss dienende (gebrauchs- oder verzehrfertige) Zubereitungen (wie oben beschrieben), die bezogen auf das Gesamtgewicht der Zubereitung eine Gesamtmenge an Bestandteil (A) im Bereich von 0,5 ppm bis 5000 ppm, vorzugsweise 5 ppm bis 1000 ppm, besonders bevorzugt 50 ppm bis 500 ppm, enthalten, sind besonders bevorzugt.

Demgegenüber weisen erfindungsgemäße Halbfertigwaren (wie oben beschrieben) bezogen auf das Gesamtgewicht der Halbfertigware vorzugsweise eine Gesamtmenge an Bestandteil (A) im Bereich von 5 ppm bis 500000 ppm, vorzugsweise 50 ppm bis 100000 ppm, besonders bevorzugt 250 ppm bis 5000 ppm, auf.

Erfindungsgemäße Halbfertigwaren, insbesondere vorstehend als bevorzugt bezeichnete Halbfertigwaren, eignen sich vorteilhafterweise besonders gut zur Herstellung von (vorzugsweise erfindungsgemäßen) der Ernährung oder dem Genuss dienenden (gebrauchs- oder verzehrfertigen) Zubereitungen.

Der Ernährung oder dem Genuss dienende Zubereitungen im Sinne der vorliegenden Erfindung sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Die erfindungsgemäßen der Ernährung oder dem Genuss dienenden Zubereitungen können im Rahmen der vorliegenden Erfindung auch im Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen oder insbesondere Nahrungsergänzungsmittel vorliegen.

Bevorzugt wird dabei eine Menge an Lactisolverbindung(en) bzw. an erfindungsgemäßer Zusammensetzung eingesetzt, die ausreicht, um eine Zubereitung bzw. Halbfertigware herzustellen, die eine wirksame Menge (siehe oben) an Lactisolverbindung(en) enthält.

Die erfindungsgemäßen Zusammensetzungen, Zubereitungen bzw. Halbfertigwaren (wie jeweils oben beschrieben) werden vorzugsweise hergestellt, indem die Lactisolverbindung(en) zunächst mit Ethanol und (gegebenenfalls demineralisiertem und/oder gereinigtem) Wasser gemischt bzw. darin gelöst werden. Anschließend werden diese Lösungen vorzugsweise durch einen Trocknungsprozess, vorzugsweise einen Sprühtrocknungs-, Vakuumgefriertrockungs-, Umkehrosmose-, Eindampf- oder einen anderen Konzentrationsprozess oder eine Kombination dieser genannten Prozesse, in eine (zumindest nahezu) feste Zusammensetzung bzw. Zubereitung oder Halbfertigware überführt. Die Trocknung kann unter Zuhilfenahme von Trägerstoffen (z.B. Stärke, Stärkederivate, Maltodextrin, Kieselgel, siehe oben) oder Hilfsstoffen (z.B. Pflanzengummen, Stabilisierungsmittel) erfolgen. Vorzugsweise erfolgt die Trocknung dabei mittels Sprühtrocknung oder Vakuumgefriertrocknung.

Weitere übliche Wirk-, Grund-, Hilfs- und Zusatzstoffe für der Ernährung oder dem Genuss dienende oder orale pharmazeutische Zubereitungen können in Mengen von 0,9 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, enthalten.

Weiterhin werden erfindungsgemäße Zubereitungen (wie oben beschrieben) besonders bevorzugt hergestellt, indem die Lactisolverbindung(en), insbesondere die Verbindungen der Formel (I) bzw. eine Mischung davon, als Substanz(en), als Lösung oder in Form eines Gemisches mit einem festen oder flüssigen Trägerstoff in eine der Ernährung oder dem Genuss dienende oder eine pharmazeutische Basis-Zubereitung (zur oralen Applikation) eingearbeitet werden. Vorteilhafterweise können als Lösung vorliegende erfindungsgemäße Zubereitungen auch durch Sprühtrocknung in eine feste Zubereitung überführt werden.

Gemäß einer weiteren bevorzugten Ausführungsform der vorliegenden Erfindung werden zur Herstellung erfindungsgemäßer Zubereitungen die Lactisolverbindungen sowie ggf. weitere Bestandteile der erfindungsgemäßen Zubereitung vorher in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten, Cellulose oder Cellulosederivaten (z.B. Hydroxypropylcellulose), anderen Polysacchariden (z.B. Alginat), natürlichen Fetten, natürlichen Wachsen (z.B. Bienenwachs, Carnaubawachs) oder aus Proteinen, z.B. Gelatine, eingearbeitet.

In einem weiteren bevorzugten Herstellungsverfahren erfindungsgemäßer Zubereitungen werden die Lactisolverbindungen vorher mit einem oder mehreren geeigneten Komplexbildnern, beispielsweise mit Cyclodextrinen oder Cyclodextrinderivaten, bevorzugt alpha- oder beta-Cyclodextrin, komplexiert und in dieser komplexierten Form eingesetzt.

Als weitere Bestandteile für erfindungsgemäße, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. gamma-Aminobuttersäure, Taurin), Peptide (z.B. Glutathion), native oder prozessierte Proteine (z.B. Gelatine), Enzyme (z.B. Peptidasen), Nukleinsäuren, Nucleotide, andere Geschmackskorrigenzien für unangenehme Geschmackseindrücke, Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), zusätzliche Bitterstoffe (z.B. Chinin, Coffein, Limonin, Amarogentin, Humolone, Lupolone, Catechine, Tannine), Süßstoffe (z.B. Saccharin, Cyclamat, Aspartam, Neotam), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Kaugummis, bevorzugte erfindungsgemäße der Ernährung oder dem Genuss dienende Zubereitungen (wie oben beschrieben), umfassen im Allgemeinen eine Kaugummibase, d.h. eine beim Kauen plastisch werdende Kaumasse, Zucker verschiedener Arten, Tauschstoffe, Süßstoffe, Zuckeralkohole, andere Geschmackskorrigenzien für unangenehme Geschmackseindrücke, Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Gemäß einem weiteren Aspekt der vorliegenden Erfindung werden die (erfindungsgemäßen) Lactisolverbindungen, insbesondere die Verbindungen der Formel (I) (wie oben beschrieben), vorzugsweise in Verbindung mit einer oder mehreren Substanzen zum Verändern, Maskieren oder Vermindern des unangenehmen Geschmackseindrucks eines unangenehmen schmeckenden Stoffes und/oder zum Verstärken eines angenehmen Geschmackseindrucks eines angenehm schmeckenden Stoffes eingesetzt, wobei diese weitere(n) Substanz(en) keine erfindungsgemäßen Lactisolverbindungen (wie oben beschrieben), insbesondere keine Verbindung(en) der Formel (I), ist bzw. sind. Dadurch werden vorteilhafterweise gegebenenfalls vorhandene Fehlnoten der (erfindungsgemäßen) Lactisolverbindungen bzw. insbesondere der Verbindungen der Formel (I) vermindert oder (zumindest teilweise) maskiert. Eine erfindungsgemäße Zubereitung oder Halbfertigware (wie oben beschrieben), die in Bestandteil (B) bzw. (C) eine oder mehrere solche Substanzen umfasset, werden somit vom Verbraucher als angenehmer und hochwertiger empfunden.

Diese Substanzen bzw. Geschmackskorrigenzien sind vorzugsweise aus der folgenden Liste ausgewählt, ohne die vorliegende Erfindung damit einzuschränken: Nucleotide (z.B. Adenosin-5'-monophosphat, Cytidin-5'-monophosphat) oder deren pharmazeutisch akzeptablen Salze, Natriumsalze (z.B. Natriumchlorid, Natriumlactat, Natriumcitrat, Natriumacetat, Natriumgluconoat), Hydroxyflavanonen, wie zum Beispiel Eriodictyol, Sterubin (Eriodictyol-7-methylether), Homoeriodictyol, und deren Natrium-, Kalium-, Calcium-, Magnesium- oder Zinksalzen (insbesondere solche wie beschrieben in EP 1 258 200, die bezüglich der darin offenbarten entsprechenden Verbindungen auf dem Wege der Verweisung Bestandteil dieser Anmeldung wird), Hydroxybenzoesäureamiden, wie zum Beispiel 2,4-Dihydroxybenzoesäurevanillylamid, 2,4-Dihydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-*N*-(4-hydroxy-3-methoxybenzyl)amid, 2,4-Dihydroxybenzoesäure-*N*-(4-hydroxy-3-methoxy-benzyl)amid-Mono-natriumsalz, 2,4-Dihydroxybenzoesäure-*N*-2-(4-hydroxy-3-methoxyphenyl)-ethylamid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-*N*-[2-(4-hydroxy-3-methoxyphenyl)ethyl]amid; 4-Hydroxybenzoesäurevanillylamid (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, wie z.B. 2-(4-Hydroxy-3-methoxyphenyl)-1-(2,4,6-trihydroxyphenyl)-ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon) (insbesondere solche wie beschrieben in WO 2006/106023, die bezüglich der darin offenbarten entsprechenden Verbindungen auf dem Wege der Verweisung Bestandteil dieser Anmeldung wird); Hydroxyphenlaalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); gamma-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird); Divanillinen (insbesondere solche wie beschrieben in WO 2004/078302, die bezüglich der darin offenbarten entsprechenden Verbindungen auf dem Wege der Verweisung Bestandteil dieser Anmeldung wird) und 4-Hydroxydihydrochalconen, vorzugsweise wie beschrieben in US 2008/227867 A1 (die bezüglich der darin offenbarten entsprechenden Verbindungen auf dem Wege der Verweisung Bestandteil dieser Anmeldung wird), dabei insbesondere Phloretin und Davidigenin, Aminosäuren oder Gemische von Molkeproteinen mit Lecithinen, Hesperitin wie in der WO 2007/014879 offenbart, die hinsichtlich dieser Verbindungen auf dem Wege der Verweisung Bestandteil dieser Anmeldung wird, 4-Hydroxydihydrochalkone wie in der WO 2007/107596 offenbart, die hinsichtlich dieser Verbindungen auf dem Wege der Verweisung Bestandteil dieser Anmeldung wird, oder Propenylphenylglycoside (Chavicolglycoside) wie in EP 1 955 601 A1 beschrieben, die hinsichtlich dieser Verbindungen auf dem Wege der Verweisung Bestandteil dieser Anmeldung wird, Deoxybenzoinen wie zum Beispiel 2-(4-Hydroxyphenyl)-1-(4-hydroxy-3-methoxyphenyl)ethanon (insbesondere solche wie beschrieben in DE 10 2009 002 268.6 und den jeweils darauf basierenden Patentanmeldungen, die bezüglich der darin offenbarten entsprechenden Verbindungen auf dem Wege der Verweisung Bestandteil dieser Anmeldung wird), Hydroxyflavanen wie zum Beispiel 3',7-Dihydroxy-4'-methoxyflavan (insbesondere solche wie beschrieben in US Provisional Application 61/178,667 und den darauf basierenden Patentanmeldungen, die bezüglich der darin offenbarten entsprechenden Verbindungen auf dem Wege der Verweisung Bestandteil dieser Anmeldung werden), Umami-Verbindungen wie die in WO 2008/046895 A1 und EP 1 989 944 A1 beschrieben, die jeweils hinsichtlich dieser Verbindungen auf dem Wege der Verweisung Bestandteil dieser Anmeldung werden, sowie Umami-Verbindungen wie beschrieben in US Provisional Application 60/984,023 bzw. US Provisional Application 61/061,273 bzw. und den jeweils darauf basierenden Patentanmeldungen, die bezüglich der darin offenbarten entsprechenden Verbindungen auf dem Wege der Verweisung Bestandteil dieser Anmeldung werden.

### VERWENDUNG

Eine weitere Ausführungsform der vorliegenden Erfindung betrifft die Verwendung von Lactisolverbindungen der Formel (I) als Süßrezeptor-Antagonisten, speziell als T1R2/T1R3-Rezeptor-Antagonisten sind.

Nachfolgend wird die Erfindung anhand von Beispielen näher erläutert. Sofern nicht anders angegeben, beziehen sich alle Angaben und Daten, insbesondere Prozent- und Mengenangaben, auf das Gewicht.

### BEISPIELE

### ALLGEMEINE ARBEITSVORSCHRIFT ZUR HERSTELLUNG VON VERBINDUNGEN DER FORMEL (I) WIE OBEN BESCHRIEBEN (AAV1):

Zu einer Lösung aus 1,0 Äquivalenten Lactisol ((2-(4-Methoxyphenoxy)propionsäure) und 5 Tropfen N-Methylformanilid in 2ml/mmol Dichlormethan werden 1,2 Äquivalente Oxalylchlorid langsam zugetropft. Die Reaktionsmischung wird anschließend für 2 Stunden bei Raumtemperatur und nochmals 3 Stunden unter Rückfluss gerührt. Nach Entfernen des überschüssigen Lösungsmittels sowie gegebenenfalls von Oxalylchlorid und HCl wird eine 1 M Lösung des Säurechlorids in Dichlormethan hergestellt.

Zu einer Mischung aus dem zur Umsetzung mit dem Säurechlorid vorgesehenen Alkohol und Triethylamin (1,1 Äquivalente pro vorhandener Alkoholfunktion) in 2 ml/mmol Dichlormethan werden 1,2 Äquivalente (pro vorhandener Alkoholfunktion) der oben dargestellten Säurechloridlösung langsam zugetropft. Nach Abklingen der exothermen Reaktion wird für weitere fünf Stunden unter Rückfluss gerührt. Anschließend wird die Reaktionsmischung mit Wasser verdünnt und die organische Phase abgetrennt.

Die organische Phase wird anschließend mit gesättigter Natriumhydrogencarbonatlösung gewaschen und über Natriumsulfat getrocknet. Nach Entfernen des Lösungsmittels und anschließender säulenchromatographischer Aufreinigung (Hexan/Essigester) werden die gewünschten (aufgereinigten) Lactisolester erhalten.

### BEISPIEL 1

### 2-(4-Methoxyphenoxy)propansäurebutylester (Verbindung der Formel (4))

4,60 g (62,1 mmol) 1-Butanol als zur Umsetzung vorgesehener Alkohol wurden nach AAV1 umgesetzt. Nach säulenchromatographischer Aufreinigung wurden 9,10 g (36,1 mmol) der Verbindung (4) als Flüssigkeit erhalten.
**¹H-NMR (400 MHz, CDCl₃):** 0.89 (t, *J* = 7.5 Hz, 3H); 1.31 (m, 2H); 1.59 (d, *J* = 6.8 Hz, 3H); 1.59 (m, 2H); 3.75 (s, 3H); 4.14 (m, 2H); 4.67 (q, *J* = 7.0 Hz, 1H); 6.78 - 6.85 (kB; 4H) ppm.
**¹³C-NMR (100 MHz, CDCl₃):** 13.6 (CH₃); 18.7 (CH₃); 19.0 (CH₂); 30.6 (CH₂); 55.6 (CH₃); 65.0 (CH₂); 73.6 (CH); 114.6 (CH); 116.4 (CH); 151.8 (C); 154.4 (C); 172.5 (C=O) ppm.
**Massenspektrum (EI):** m/z (%) = 252 (M^{•+}, 84); 151 (86); 124 (87); 123 (100); 109 (42); 92 (20); 77 (26); 41 (22); 29 (24); 28 (61).

### BEISPIEL 2

### 2-(4-Methoxyphenoxy)-propansäurehexylester (Verbindung der Formel (1))

6,40 g (62,6 mmol) Hexanol wurden nach AAV1 umgesetzt. Nach säulenchromatographischer Aufreinigung wurden 10,20 g (36,4 mmol) der Verbindung (1) als Flüssigkeit erhalten.
**¹H-NMR (400 MHz, CDCl₃):** 0.77 (t, *J* = 6.7 Hz, 3H); 1.24 - 1.29 (kB, 6H); 1.59 (d, *J* = 6.7 Hz, 3H); 1.59 (m, 2H); 3.75 (s, 3H); 4.14 (m, 2H); 4.66 (q, *J* = 6.9 Hz, 1H); 6.78 - 6.85 (kB; 4H) ppm.
**¹³C-NMR (100 MHz, CDCl₃):** 14.0 (CH₃); 18.7 (CH₃); 22.5 (CH₂); 25.4 (CH₂); 28.5 (CH₂); 31.4 (CH₂); 55.6 (CH₃); 65.3 (CH₂); 73.6 (CH); 114.6 (CH); 116.4 (CH); 151.8 (C); 154.4 (C); 172.6 (C=O) ppm.
**Massenspektrum (EI):** m/z (%) = 281 (18); 280 (M^{•+}, 88); 151 (90); 124 (92); 123 (100); 109 (32); 77 (25); 43 (37); 41 (26); 28 (64).

### BEISPIEL 3

### 2-(4-Methoxyphenoxy)propansäure-(E)-hex-2-enylester

3,30 g (32,9 mmol) (E)-2-Hexen-1-ol wurden nach AAV1 umgesetzt. Nach säulenchromatographischer Aufreinigung wurden 4,20 g (15,1 mmol) der gewünschten Verbindung als Flüssigkeit erhalten.
**¹H-NMR (400 MHz, CDCl₃):** 0.88 (t, *J* = 7.4 Hz, 3H); 1.38 (hex, *J* = 7.4, 2H); 1.59 (d, *J* = 6.8 Hz, 3H); 2.01 (m, 2H); 3.75 (s, 3H); 4.58 (m, 2H); 4.66 (q, *J* = 6.8 Hz, 1H); 5.52 (m, 1H); 5.74 (m, 1H); 6.78 - 6.85 (kB; 4H) ppm.
**¹³C-NMR (100 MHz, CDCl₃):** 13.6 (CH₃); 18.6 (CH₃); 22.0 (CH₂); 34.3 (CH₂); 55.6 (CH₃); 65.8 (CH₂); 73.6 (CH); 114.6 (CH); 116.4 (CH); 123.4 (CH); 137.0 (CH); 151.7 (C); 154.5 (C); 172.2 (C=O) ppm.
**Massenspektrum (EI):** m/z (%) = 278 (M^{•+}, 71); 151 (100); 124 (43); 123 (58); 119 (32); 109 (17); 77 (24); 55 (47); 41 (30); 28 (21).

### BEISPIEL 4

### 2-(4-Methoxyphenoxy)-propansäure-2-isopropyl-5-methyl-phenylester

4,96 g (32,0 mmol) Thymol wurden nach AAV1 umgesetzt. Nach säulenchromatographischer Aufreinigung wurden 5,24 g (16,0 mmol) der gewünschten Verbindung als Flüssigkeit erhalten.
**¹H-NMR (400 MHz, CDCl₃):** 1.07 (d, *J* = 6.9 Hz, 3H); 1.11 (d, *J* = 6.9, 3H); 1.77 (d, *J* = 6.8 Hz, 3H); 2.27 (s, 3H); 2.77 (hept, J = 6.9 Hz, 1H); 3.75 (s, 3H); 4.93 (q, *J* = 6.8 Hz, 1H); 6.69 - 7.01 (kB, 7H) ppm.
**¹³C-NMR (100 MHz, CDCl₃):** 18.7 (CH₃); 20.8 (CH₃); 22.9 (CH₃); 23.0 (CH₃); 26.7 (CH); 55.65 (CH₃); 73.7 (CH); 114.7 (CH); 116.4 (CH); 122.2 (CH); 126.4 (CH); 127.4 (CH); 136.6 (C); 137.0 (C); 147.4 (C); 151.7 (C); 154.7 (C); 171.3 (C=O) ppm.
**Massenspektrum (EI):** m/z (%) = 328 (M^{•+}, 32); 177 (24); 151 (48); 150 (24); 135 (13); 124 (100); 123 (14); 119 (23); 91 (14); 77 (22).

### BEISPIEL 5

### 2-(4-Methoxyphenoxy)propansäure-2-[2-(4-methoxyphenoxy)-propionyloxy]-ethylester (Verbindung der Formel (3))

1,03 g (16,6 mmol) Glycol wurden nach AAV1 umgesetzt. Nach säulenchromatographischer Aufreinigung wurden 5,50 g (13,1 mmol) der Verbindung (3) als leicht viskose Flüssigkeit erhalten.
**¹H-NMR (400 MHz, CDCl₃):** 1.555 (d, *J* = 6.8 Hz, 3H); 1.557 (d, *J* = 6.8 Hz, 3H); 3.738 (s, 3H); 3.740 (s, 3H); 4.29 - 4.41 (kB, 4H); 4.63 (q, *J* = 6.8 Hz, 1H); 4.64 (q, *J* = 6.8 Hz, 1H); 6.77 - 6.85 (kB, 8H) ppm.
**¹³C-NMR (100 MHz, CDCl₃):** 18.5 (CH₃); 55.6 (CH₃); 62.5 (CH₂); 73.4 (CH); 73.5 (CH); 114.7 (CH); 116.5 (CH); 116.6 (CH); 151.6 (C); 154.5 (C); 154.6 (C); 172.12 (C=O); 172.13 (C=O) ppm.
**Massenspektrum (EI):** m/z (%) = 419 (29); 418 (M^{•+}, 84); 223 (38); 177 (35); 151 (82); 124 (54); 123 (100); 99 (26); 77 (31); 28 (55).

### BEISPIEL 6

### 2-(4-Methoxyphenoxy)propansäure-2,3-bis-[2-(4-methoxyphenoxy)-propionyloxy]-propylester (Verbindung der Formel (2))

1,04 g (11,1 mmol) Glycerin wurden nach AAV1 umgesetzt. Nach säulenchromatographischer Aufreinigung wurden 6,0 g (9,6 mmol) der Verbindung (2) als viskose Flüssigkeit erhalten.
**¹H-NMR (400 MHz, CDCl₃):** 1.50 - 1.56 (kB, 9H); 3.72 - 3.75 (kB, 9H); 3.99 - 4.13 (kB, 2H); 4.26 - 4.32 (kB, 1H); 4.37 - 4.43 (kB, 1H); 4.54 - 4.69 (kB, 3H); 5.27 (m, 1H); 6.78 -6.81 (kB, 12H) ppm.
**¹³C-NMR (100 MHz, CDCl₃):** 18.44 (CH₃); 18.46 (CH₃); 18.50 (CH₃); 18.51 (CH₃); 18.54 (CH₃); 55.63 (CH₃); 55.65 (CH₃); 62.29 (CH₂); 62.34 (CH₂); 62.38 (CH₂); 69.55 (CH); 69.57 (CH); 73.12 (CH); 73.17 (CH); 73.23 (CH); 73.28 (CH); 73.33 (CH); 114.69 (CH); 114.74 (CH); 116.36 (CH); 116.39 (CH); 116.40 (CH); 116.42 (CH); 116.43f (CH); 116.47 (CH); 116.49 (CH); 151.48 (C); 151.49 (C); 154.56 (C); 154.58 (C); 154.60 (C); 154.61 (C); 171.42 (C=O); 171.44 (C=O); 171.45 (C=O); 171.48 (C=O); 171.72 (C=O); 171.75 (C=O); 171.77 (C=O); 171.79 (C=O); 171.81 (C=O) ppm.
**Massenspektrum** (LC-MS: *C18; 100x2.1 mm; 0.2 ml*/*min; H₂O*/*CH₃CN 100*/*0* → *0*/*100, 15 min*.): m/z (%) = 646 (6); 645 (100); 433 (13); 432 (14); 431 (7) 430 (7).

### ANWENDUNGSBEISPIEL 1

### Einfluss von Verbindungen der Formel (I) (wie oben beschrieben) auf die sensorische Qualität von süßen Stoffen

Um den Einfluss erfindungsgemäßer Verbindungen der Formel (I) auf die sensorischen Eigenschaften unterschiedlicher süßer Stoffe zu quantifizieren, wurden jeweils eine Probe (A) (süßer Stoff) und eine Probe (B) (süßer Stoff + Verbindung der Formel (I) bzw. Lactisol (als Standard)) von einer Expertengruppe sensorisch beurteilt und entsprechend ihres Süß-Eindrucks anhand einer Skala von 1 [nicht süß] bis 10 [extrem süß] bewertet.

Anschließend wurde jeweils aus den Durchschnittswerten der Bewertungen der Proben (A) und (B) die Veränderung (in %) des Süß-Eindrucks ermittelt. Die Ergebnisse sind in der nachfolgenden **Tabelle 1** zusammengefasst:

**Tabelle 1**

| Veränderung des Süßeindrucks | | | | |
|---|---|---|---|---|
| **Verbindung der Formel (I) bzw. Lactisol/Konzentration** | **Süßer Stoff / Konzentration** | **Süß-Eindruck (1-10)** | | **Veränderung des Süß-Eindrucks [%]** |
| | | **Probe A** | **Probe B** | |
| Lactisol / 10 mg/l | Sucrose / 5 % | 5,8 ± 1,2 | 4,2 ± 1,4 | -27 % (p < 0,005) |
| Verbindung der Formel (1), aus Beispiel 2 / 14 mg/l | Sucrose / 5 % | 5,6 ± 1,4 | 5,0 ± 1 | -10 % (nicht signifikant) |
| Verbindung der Formel (2), aus Beispiel 6 / 10 mg/l | Sucrose / 5 % | 5,4 ± 1,5 | 5,6 ± 1,5 | 3 % (nicht signifikant) |
| Verbindung der Formel (2), aus Beispiel 6 / 10 mg/l | Naringindihydrochalkon / 50 mg/l | 5,1 ± 2,1 | 5,6 ± 2,1 | 9 % (nicht signifikant) |
| Verbindung der Formel (4), aus Beispiel 1 / 16 mg/l | Sucrose / 5 % | 5,8 ± 1.1 | 5,6 ± 1,5 | -5 % (nicht signifikant) |

Dabei ist erkennbar, dass die erfindungsgemäßen Verbindungen der Formel (I) im Gegensatz zum bekannten Süßinhibitor Lactisol keinen signifikanten Einfluss auf die Süßwirkung der getesteten Stoffe haben.

### ANWENDUNGSBEISPIEL 2

### Freisetzung von Lactisol aus Verbindungen der Formel (I) bei der künstlichen Verdauung

Untersucht wurde die Freisetzung von Lactose aus den gemäß den Beispielen 1, 2, 5 und 6 hergestellten Verbindungen der Formeln (1), (2), (3) und (4) im Verlauf einer künstlichen Verdauung. Lactisol diente als (Standard-)Referenz und wurde in analoger Weise einer künstlichen Verdauung unterzogen.

Unter dem Begriff "künstliche Verdauung" wird in diesem Zusammenhang folgende Verfahrensweise verstanden: 1,0 g der zu untersuchenden Verbindung wird in 6,25 g Kunstspeichel [5,0 g Kaliumhydrogencarbonat; 0,64 g Kaliumchlorid; 0,15 g Natriumcarbonat; 206,18 mg alpha-Amylase (48,5 Units); 100 mg Mucin; destilliertes Wasser ad 1000 ml] für 15 Minuten bei 37 °C stark gerührt.

Danach wird eine (erste) Probe ("Mund", siehe unten) entnommen, und die (restliche) Mischung wird mit 10%iger Salzsäure auf pH 2.5 eingestellt und mit 20 ml künstlichem Magensaft [0.88 g Natriumchlorid; 500 mg Pepsin; destilliertes Wasser ad 100 ml; pH 2.5, eingestellt mit 10%iger Salzsäure] für weitere 4 Stunden bei 37 °C stark gerührt.

Danach wird eine (zweite) Probe ("Magen", siehe unten) entnommen und die (restliche) Mischung wird mit 10%iger Natronlauge auf pH 7.5 eingestellt und mit 10 ml künstlichem Darmsaft [30 mg Kaliumchlorid; 50 mg Kalziumchlorid; 20 mg Magnesiumchlorid; 100 mg Natriumcarbonat; 900 mg Pankreatin; 900 mg Galle, lyophilisiert; destilliertes Wasser ad 100 ml; pH 7.5, eingestellt mit 10%iger Natronlauge oder 10%iger Salzsäure] für weitere 3 Stunden bei 37 °C heftig gerührt.

Danach wird eine (dritte) Probe ("Darm", siehe unten) entnommen.

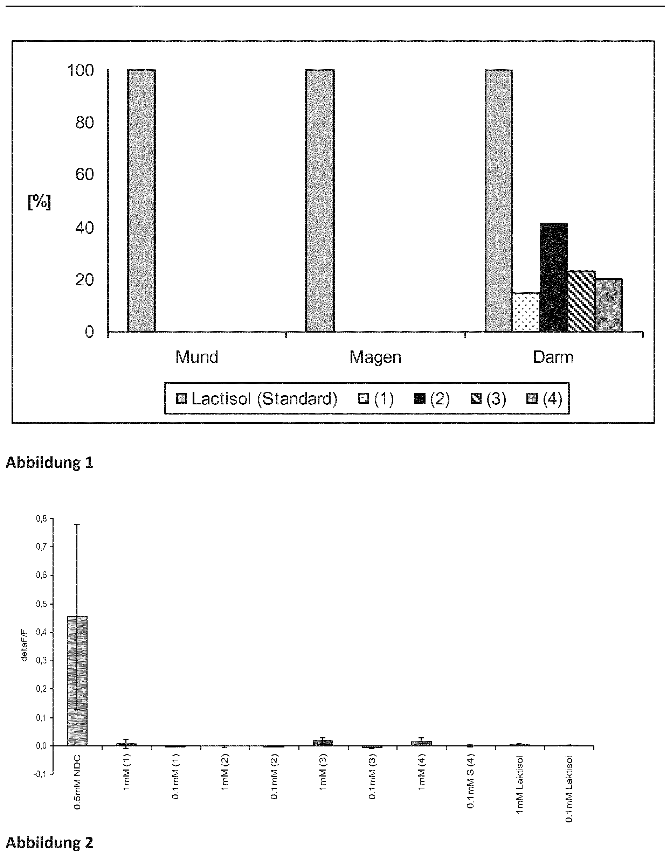

Die entnommen (drei) Proben werden dann jeweils via LC-MS untersucht. Zur Quantifizierung von (freigesetztem) Lactisol wurde die UV-Spur benutzt. Die Ergebnisse sind in der **Abbildung 1** wiedergegeben. Diese zeigt die aus den Verbindungen der Formeln (1), (2), (3) und (4) im Verlauf der künstlichen Verdauung freigesetzte Menge an Lactisol. Dabei wurde die gemessene Menge an Lactisol zu der theoretisch maximal zu erwartenden freigesetzten Menge - je Anzahl der mit Lactisol veresterten Alkoholfunktionen - ins Verhältnis gesetzt. In der Abbildung ist zu erkennen, dass von den Verbindungen der Formeln (1) bis (4) unter den (künstlichen) Milieu-Bedingungen des Mundraums ("Mund") kein Lactisol freigesetzt wurde.

Dieses Ergebnis wurde in einer weiteren Untersuchung anhand von Geschmackstests durch ein sensorisches Panel bestätigt: Bei der Verkostung der Verbindungen der Formeln (1) bis (4) wurde keine signifikante Beeinflussung der Süßintensität (im Mundraum) festgestellt, während bei der Verkostung von Lactisol erwartungsgemäß eine deutliche Verminderung der Süßintensität bemerkt wurde. Dies lässt darauf schließen, dass bei der Verkostung der Verbindungen der Formeln (1) bis (4) im Mundraum kein Lactisol freigesetzt wurde.

In Abbildung 1 ist ebenfalls zu erkennen, dass von den Verbindungen der Formeln (1) bis (4) auch unter den (künstlichen) Milieu-Bedingungen der Magenpassage ("Magen") kein Lactisol freigesetzt wurde.

Nach Zugabe des künstlichen Darmsaftes (und 3 Stunden heftigem Rühren bei 37 °C, siehe oben) konnte dagegen eine Freisetzung von Lactisol aus den Verbindungen der Formeln (1) bis (4) beobachtet werden (siehe Diagramm1, "Darm"). Zudem kann den Werten entnommen werden, dass die Verbindung der Formel (2) für die Zwecke der vorliegenden Erfindung besonders geeignet ist.

Die Untersuchungsergebnisse bestätigen die erfindungsgemäße Anwendung von Verbindungen der Formel (I) zur (gezielten) Beeinflussung der Süßrezeptoren im Darm, ohne (bei oraler Gabe) den Süßgeschmack im Mundraum zu beeinträchtigen.

### ANWENDUNGSBEISPIEL 3

### Untersuchungen zur aktivierenden oder inhibierenden Wirkung der Verbindungen der Formeln (1) bis (4) auf T1R2/T1R3-Rezeptoren

Eine aus humanen, embryonalen Nierenzellen abgeleitete, stabile Zelllinie (HEK293TREX) wurde mit Hilfe des Plasmids plRESpuro3 hergestellt, welches die G-Protein-Chimäre G15-Gi3 kodiert. Ein weiteres Plasmid (pCDNA5-FRT), welches die humane TAS1R2-Sequenz kodiert, wurde zusätzlich benutzt. Dadurch war es möglich, das Gen an einer definierten chromosomalen Region zu integrieren. Die menschliche TAS1R3 Untereinheit des humanen Süßrezeptors wurde in pCDNA3-TO kloniert. Dies ermöglicht die Induktion des Zielgens mittels Tetracyclin. Die stabile, transfizierte Zelllinie wurde in hoch-Glucose-haltigem DMEM ohne Tetracyclin kultiviert. Für die Messungen wurden die Zellen in Polylysin-beschichteten 96-Well-Mikrotiterplatten (schwarz mit transparentem Boden) gesät und kultiviert (37 °C, 5 % CO₂) bis sie 80 % Konfluenz erreichten. 24 h vor den eigentlichen Messungen wurden die Zellen in niedrig-Glucose-haltigem DMEM, GlutaMAX, 10% dialysiertem FBS (Invitrogen, Carlsbad, CA) und Tetracyclin inkubiert, um die Expression des TAS1R3 zu induzieren. Eine Kontrollprobe von Zellen wurde nicht mit Tetracyclin versetzt (mock-Zellen). Ungefähr 2 h vor der Messung wurden die Zellen 1 h mit dem Calciumempfindlichen Farbstoff Fluo4- AM (2 µg/ml in DMEM, Molecular Probes, Carlsbad, CA) behandelt. Die Zellen wurden danach dreimal mit einer Lösung C1 (130 mM NaCl, 5 mM KCl, 10 mM Hepes, 2 mM CaCl₂, and 5 mM Glucose, pH 7,4) gewaschen. Die Calcium-Mobilisierung durch Rezeptor-Stimulation durch die Testsubstanzen wurde in einem automatisierten Fluorometer gemessen (fluorometric imaging plate reader FLIPR, Molecular Devices, Sunnyvale, CA). Die Testsubstanzen wurden in einer Konzentration von 1 mmol/L bzw. 0.1 mmol/L in C1-Lösung gemessen. Alle Daten wurden in zwei unabhängigen Experimenten bestimmt, die jeweils dreimal wiederholt wurden. Die erhaltenen Calciumsignale wurden um den Anteil der mock-Zellen korrigiert und (unter Verwendung der Formel ΔF/F = (F-F₀)/F₀) auf die Anfangs-Intensität der Fluoreszenz vor Zugabe der Testsubstanzen (F₀) normalisiert.

**Abbildung 2** zeigt die Aktivierung heterolog exprimierter Süßrezeptoren TAS1R2/ TAS1R3 durch die Verbindungen der Formeln (1) bis (4) im Vergleich zu Naringindihydrocha-Ikon (NDC) und Lactisol.

**Abbildung 3** zeigt die Beeinflussung der durch Naringindihydrochalkon (NDC) verursachten Aktivierung des heterolog exprimierten Süßrezeptors TAS1R2/TAS1R3 durch die Verbindungen der Formeln (1) und (2).

In Abbildung 2 ist erkennbar, dass die erfindungsgemäßen Verbindungen (wie auch Lactisol) keinen nennenswerten agonistischen Effekt auf den Süßrezeptor ausüben.

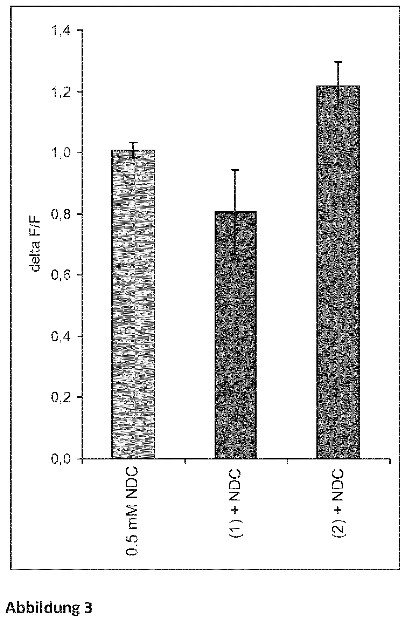

In Abbildung 3 ist erkennbar, dass die erfindungsgemäßen Verbindungen der Formeln (1) und (2)- im Gegensatz zu Lactisol - selbst keinen antagonistischen Effekt (in Gegenwart des beispielhaften süßen Stoffes Naringindihydrochalkon) zeigen. Dies bestätigt die Einsatzmöglichkeit der erfindungsgemäßen Verbindungen der Formel (I) für eine selektiv im (Dünn-)Darm gewünschte antagonistische Wirkung ohne die Süßrezeptoren im Mundraum zu inhibieren.

### ANWENDUNGSBEISPIEL 4

### Sprühgetrocknete Zusammensetzung

| **Inhaltsstoff** | **Anteil [g]** |
|---|---|
| Verbindung der Formel (2) | 4 |
| Maltodextrin | 96 |

Die beiden Bestandteile werden in einer Mischung aus Ethanol und demineralisiertem Wasser gelöst und anschließend sprühgetrocknet.

### ANWENDUNGSBEISPIEL 5

### Aromakomposition

| **Inhaltsstoff** | **Anteil [g]** |
|---|---|
| 10 Gew.-% Pellitorin in 1,2-Propylenglycol/Diethylmalonat | 0,25 |
| Hesperetin | 2,50 |
| Phloretin | 1,50 |
| Verbindung der Formel (2) | 1,50 |
| Propylenglycol | 94,25 |

Die Aromakomposition wurde in nachfolgend beschriebenen Anwendungsbeispielen eingesetzt.

### ANWENDUNGSBEISPIEL 6

### Zuckerfreier Kaugummi

| **Teil** | **Inhaltsstoff** | **Menge [Gew.-%]** |
|---|---|---|
| A | Kaugummibase, Company "Jagum T" | 30,00 |
| B | Sorbit, pulverisiert | 39,00 |
| | Isomalt® (Palatinit GmbH) | 9,50 |
| | Xylit | 2,00 |
| | Mannit | 3,00 |
| | Aspartam® | 0,10 |
| | Acesulfam® K | 0,10 |
| | Emulgum® (Colloides Naturels, Inc.) | 0,30 |
| C | Sorbitol, 70 % | 14,00 |
| | Glycerin | 1,00 |
| D | Aromakomposition, gemäß Anwendungsbeispiel 5 | 1,00 |

Die Teile A bis D werden gemischt und intensiv geknetet. Die erhaltene Rohmasse kann dann z.B. in Form von dünnen Streifen zu verzehrfertigen Kaugummis verarbeitet werden.

### ANWENDUNGBEISPIEL 7

### Zuckerfreie Hartkaramelle

| **Inhaltsstoff** | **Menge [Gew.-%]** |
|---|---|
| Palatinit, Typ M | 75,07 |
| Wasser | 24,82 |
| Pfefferminzaroma | 0,1 |
| Verbindung der Formel (2) | 0,01 |

Zunächst wurde Palatinit mit Wasser gemischt. Die Mischung wurde dann bei 165 °C aufgeschmolzen und anschließend auf 115°C abgekühlt. Dann wurden das Pfefferminzaroma und die Verbindung der Formel (2) zugegeben. Nach dem Durchmischen wurden die Mischungen in Formen gegossen, nach dem Erstarren aus den Formen entfernt und anschließend einzeln verpackt.

### ANWENDUNGSBEISPIEL 8

### Schokolade

A = dunkle Schokolade
B = kalorienreduzierte dunkle Schokolade
C = kalorienreduzierte dunkle Schokolade
D = kalorienreduzierte dunkle Schokolade
E = kalorienreduzierte Vollmilchschokolade

Mengenangaben als Gew.-%.

| **Inhaltsstoff** | | **A** | **B** | **C** | **D** | **E** |
|---|---|---|---|---|---|---|
| | Kakaobutter | 13,50 | 13,00 | 13,50 | 9,48 | 14,00 |
| A | Cacaomasse | 42,00 | 39,00 | 42,00 | 44,00 | 23,00 |
| | Erythritol | - | 47,45 | - | - | - |
| | Maltitol, kristallin | - | - | - | 23,00 | |
| | Inulin | - | - | - | 23,00 | |
| | Sorbitol | - | - | 44,00 | - | - |
| | Lactitol | - | - | - | - | 38,55 |
| | Polydextrose | - | - | - | - | 9,70 |
| | Vollmilchpulver | - | - | - | - | 14,0 |
| | Sucrose | 43,98 | - | - | - | - |
| B | Lecitin | 0,48 | 0,48 | 0,40 | 0,48 | 0,50 |
| | Vanillin | 0,02 | 0,02 | 0,02 | 0,02 | 0,20 |
| | Aspartam | - | 0,03 | 0,06 | - | 0,03 |
| | Verbindung der Formel (2) | 0,02 | 0,02 | 0,02 | 0,02 | 0,02 |

Die Inhaltsstoffe von Bestandteil A werden mit 3 - 25 % der eingesetzten Gesamtmenge an Kakaobutter (siehe oben, 13,50 Gew.-%) bei 30 bis 40 °C für 15 Minuten gemischt und verknetet. Anschließend wird Bestandteil A mit einer vorzugsweise gekühlten Walze bearbeitet, dann für weitere 18 bis 25 Stunden bei 50 bis 80 °C conchiert, wobei die restliche Kakaobutter hinzugefügt wird. Kurz vor Ende des Conchiervorgangs (1 bis 3 Stunden) werden die Inhaltsstoffe von Bestandteil B zugemischt. Nach Abkühlen auf 28 bis 31 °C wird die Masse in Form gegossen und anschließend vollständig abgekühlt.

### ANWENDUNGSBEISPIEL 9

### Kohlensäurehaltiges Getränk (Geschmacksrichtung: Cola)

A = zuckerhaltiges Getränk
B = kalorienreduziertes Getränk
C = kalorienreduziertes Getränk
D = kalorienreduziertes Getränk
E = kalorienreduziertes Getränk

Mengenangaben als Gew.-%

| **Inhaltsstoff** | **A** | **B** | **C** | **D** | **E** |
|---|---|---|---|---|---|
| Phosphorsäure 85% | 0,635 | 0,635 | 0,635 | 0,635 | 0,635 |
| Zitronensäure, wasserfrei | 0,064 | 0,064 | 0,064 | 0,064 | 0,064 |
| Koffein | 0,064 | 0,064 | 0,064 | 0,064 | 0,064 |
| Succrose | 63,600 | - | - | - | 12,9 |
| Sucralose | - | 0,126 | - | - | - |
| Erythritol | - | - | 6,000 | - | - |
| Aspartam | - | - | 0,350 | - | 0,07 |
| Steviosid | - | - | - | 0,300 | - |
| Acesulfam K | - | - | - | - | 0,07 |
| Zuckercoloeur | 0,800 | 0,800 | 0,800 | 0,800 | 0,800 |
| Getränke-Emulsion Typ: Cola | 1,445 | 1,445 | 1,445 | 1,445 | 1,445 |
| Natriumbenzoat | 0,106 | 0,106 | 0,106 | 0,106 | 0,106 |
| Verbindung der Formel (2) | 0,030 | 0,030 | 0,030 | 0,030 | 0,030 |
| Wasser | ad 100 | ad 100 | ad 100 | ad 100 | ad 100 |

Die festen Bestandteile bzw. Inhaltsstoffe werden einzeln mit Wasser vermischt, zusammengegeben und mit Wasser auf 100 g aufgefüllt. Anschließend lässt man das erhaltene Konzentrat über Nacht bei Raumtemperatur altern. Schließlich wird 1 Teil Konzentrat mit 5 Teilen kohlensäurehaltigem Wasser gemischt, in Flaschen gefüllt und verschlossen.

### ANWENDUNGSBEISPIEL 10

### Himbeermarmelade (für Diabetiker geeignet)

| **Inhaltsstoffe** | **Menge [g]** |
|---|---|
| Fruktose, kristallin | 485,0 |
| Pektin | 6,5 |
| Natriumcitrat | 1,0 |
| Wasser | 250,0 |
| Himbeeren | 500,0 |
| Kalziumlactat (3%ige Lösung) | 24 |
| Kaliumsorbat (20%ige Lösung) | 5 |
| Verbindung der Formel (2) | 0,1 |

30 g Fructose werden mit dem Pektin und dem Natriumcitrat zuerst trocken und erneut nach Zugabe von Wasser vermischt. Dann werden die Himbeeren zugegeben und die Mischung zum Sieden erhitzt. Anschließend werden die restliche 455,0 g Fructose sowie das Kalziumlactat zugegeben. Die Mischung wird auf ein Gewicht von 1000 g eingekocht, unter starkem Rühren etwas abgekühlt und mit Kalziumsorbat sowie der Verbindung der Formel (2) versetzt. Anschließend wird die Masse bei einer Temperatur von 80 °C in Gläser gefüllt.

### ANWENDUNGSBEISPIEL 11

### Kochpudding

A = zuckerhaltiger Kochpudding
B = zuckerreduzierter Kochpudding

Mengenangaben als Gew.-%

| **Inhaltsstoff** | **A** | **B** |
|---|---|---|
| Sucrose | 7,8 | 5,4 |
| Stärke | 3,0 | 3,0 |
| Magermilchpulver | 1,5 | 1,5 |
| Aubygel MR50 | 0,5 | 0,5 |
| Vanilleschoten-Extrakt, sprühgetrocknet, Symrise | 0,1 | 0,1 |
| D-Tagatose | - | 0,1 |
| Symsweet HT 40% auf Maltodextrin | - | 0,04 |
| Verbindung der Formel (2) | 0,005 | 0,005 |
| Milch, 1,5 % Fettanteil | ad 100 | ad 100 |

Die festen Inhaltsstoffe wurden vorgelegt und mit der Milch aufgerührt. Die Mischung wurde auf 95 °C für 2 min unter gutem Rühren aufgewärmt, abgefüllt und auf 5 bis 8 °C abgekühlt.

### ANWENDUNGSBEISPIEL 12

### Tafelsüße (Tabletop Sweetener)

A = zuckerhaltige Tafelsüße
B = kalorienreduzierte Tafelsüße
C = zuckerfreie Tafelsüße

Mengenangaben als Gew.-%

| **Inhaltsstoff** | **A** | **B** | **C** |
|---|---|---|---|
| Sucrose | 98,75 | 48,75 | - |
| Fruktose | - | 49,4 | - |
| Aspartam | | 0,3 | |
| Aspartam (sprühgetrocknet, 30% auf Maltodextrin) | - | - | 5,0 |
| Acesulfam | - | 0,3 | - |
| Maltodextrin | - | - | 93,75 |
| Sprühgetrocknete Zusammensetzung gemäß Anwendungsbeispiel 4 | 1,25 | 1, 25 | 1,25 |

Sämtliche Inhaltsstoffe werden miteinander gemischt und zum Schutz vor Feuchtigkeit luftdicht verpackt.

### ANWENDUNGSBEISPIEL 13

### Speiseeis Typ Vanille

A = Speiseeis (voller Zucker- und Fettgehalt)
B, C, D, E = Speiseeis mit niedrigem bzw. reduziertem glycemischen Index

Mengenangaben als Gew.-%

| **Inhaltsstoff** | **A** | **B** | **C** | **D** | **E** |
|---|---|---|---|---|---|
| Maltitol | - | 10,0 | 10,0 | - | - |
| Sucrose | 16,0 | - | - | 10,0 | - |
| Fructose | - | - | - | - | 10,0 |
| Inulin | - | 6,7 | 6,7 | 6,7 | 6,7 |
| Magermilchpulver | 12,0 | 3,68 | - | 3,68 | - |
| Milcheiweiß, Lactose < 0,1% (Alaplex 1380 PPV; Frontera) | - | 3,68 | 7,36 | 3,68 | 7,36 |
| Sahne (36% Fett) | 25,0 | - | - | - | - |
| Kokosnussöl | - | 9,0 | 9,0 | 5,0 | 9,0 |
| Emulgator (Grinsted HP 60, Danisco) | - | 0,285 | 0,285 | 0,285 | 0,285 |
| Emulgator (Cremodan 709VEG, Danisco) | 0,5 | - | - | - | - |
| Guarkernmehl | - | 0,0625 | 0,0625 | 0,0625 | 0,0625 |
| Johannisbrotkernmehl | - | 0,145 | 0,145 | 0,145 | 0,145 |
| Carragenan | 0,0175 | 0,0175 | 0,0175 | 0,0175 | 0,0175 |
| Vanillin | 0,011 | 0,011 | 0,011 | 0,011 | 0,011 |
| Aroma: Typ Vanille | 0,16 | 0,16 | 0,16 | 0,16 | 0,16 |
| beta-Carotin 30% | 0,0042 | 0,0042 | 0,0042 | 0,0042 | 0,0042 |
| Verbindung der Formel (2) | 0,01 | 0,01 | 0,01 | 0,01 | 0,01 |
| Wasser | ad 100 | ad 100 | ad 100 | ad 100 | ad 100 |

Für Beispiel A werden alle Inhaltsstoffe bei 60 °C gemischt. Für die Beispiele B bis E werden zuerst alle Bestandteile bis auf das Öl und den Emulgator bei 60 °C gemischt. Dann wird der Emulgator mit dem Öl vermischt und anschließend mit der Restmenge bei 60 °C gemischt. Nun wird die Mischung homogenisiert (150 bar / 70 °C) und anschließend pasteurisiert (83°C / 20s), bevor Sie auf 4°C abgekühlt wird. Bei dieser Temperatur wird die Mischung nun für 5 Stunden gealtert, bevor sie bei -7°C gefroren wird. Anschließend wird die Masse abgefüllt, für 2 Stunden bei -30°C schockgefroren und bei -25°C gelagert.

### ANWENDUNGSBEISPIEL 14

### Süßstoff-Tabletten (64 mg)

| **Inhaltsstoffe** | **Menge [Gew.-%]** |
|---|---|
| Natriumcyclamat | 66,59 |
| Saccharin, Natriumsalz | 7,35 |
| Neohesperidindihydrochalcon | 0,11 |
| Lactose | 20,95 |
| Carboxymethylcellulose, quervernetzt | 3,5 |
| Sprühgetrocknete Zusammensetzung nach Anwendungsbeispiel 4 | 1,5 |

Die Inhaltsstoffe werden vermischt, in Tablettenform gepresst und verpackt.

### ANWENDUNGSBEISPIEL 15

### Rosinenkekse

A = ohne Zuckergehalt
B = voller Zuckerzusatz

Mengenangabe als Gew.-%

| **Inhaltsstoffe** | | **A** | **B** |
|---|---|---|---|
| A | Margarine | 11,27 | 11,27 |
| | Magermilchpulver | 1,71 | 1,71 |
| | Sucralose | 0,034 | - |
| | Polydextrose | 24,21 | - |
| | Sucrose | - | 24,244 |
| | Salz | 0,550 | 0,550 |
| B | Vollei | 9,370 | 9,370 |
| C | Mehl | 28,32 | 28,32 |
| | Stärke | 6,83 | 6,83 |
| | Natriumhydrogencarbonat | 0,376 | 0,376 |
| | Sprühgetrocknete Zusammensetzung gemäß Anwendungsbeispiel 4 | 0,25 | 0,25 |
| D | Rosinen | 17,08 | 17,08 |

Die Inhaltsstoffe A werden 2 Minuten lang miteinander vermischt. Nach Zugabe des Volleis wird die Masse für 3 Minuten lang aufgeschlagen, bevor die Inhaltsstoffe C portionsweise untergemischt werden. Zum Schluss werden die Rosinen untergemischt. Dann werden die Kekse portioniert und bei 220 °C für 10 Minuten gebacken. Nach dem Auskühlen werden die Kekse luftdicht verpackt.

### ANWENDUNGSBEISPIEL 16

### Tomatenketchup

A = Tomatenketchup (voller Zuckergehalt)
B = zuckerreduziertes Tomatenketchup
C = zuckerreduziertes Tomatenketchup

Mengenangabe in kg.

| **Inhaltsstoff** | **A** | **B** | **C** |
|---|---|---|---|
| Tomatenpüree | 100,000 | 100,000 | 100,000 |
| Essig | 8,000 | 8,000 | 8,000 |
| Zucker | 15,000 | 11,500 | 5,000 |
| Glycyrrhizin | - | - | 0,014 |
| Hesperetin 2,5%ig in 1,2-Propylenglycol | - | 0,235 | - |
| Phloretin 2,5%ig in 1,2-Propylenglycol | - | 0,235 | - |
| Salz | 2,200 | 2,200 | 2,200 |
| Mononatriumglutamat | 0,040 | 0,040 | 0,040 |
| Zwiebel, püriert | 2,000 | 2,000 | 2,000 |
| Knoblauch, püriert | 0,100 | 0,100 | 0,100 |
| Zimtpulver | 0,030 | 0,030 | 0,030 |
| Nelkenpulver | 0,030 | 0,030 | 0,030 |
| Pfeffer (weiß), gemahlen | 0,040 | 0,040 | 0,040 |
| Pfeffer (rot), gemahlen | 0,020 | 0,020 | 0,020 |
| Muskatblüte | 0,005 | 0,005 | 0,005 |
| Verbindung der Formel (2) | 0,050 | 0,050 | 0,050 |

Die Inhaltsstoffe werden in der angegebenen Reihenfolge gemischt und das fertige Ketchup mit Hilfe eines Rührwerkes homogenisiert, in Flaschen abgefüllt und sterilisiert.

### ANWENDUNGSBEISPIEL 17

### Schokoladenkuchen

A = Schokoladenkuchen (voller Zuckergehalt)
B = zuckerreduzierter Schokoladenkuchen

Mengenangaben in g.

| **Inhaltsstoffe** | **A** | **B** |
|---|---|---|
| Sucrose | 180,0 | - |
| Lactitol Monohydrat | - | 179,5 |
| Butter | 180,0 | 180,0 |
| Vollei | 180,0 | 180,0 |
| Mehl | 150,0 | 150,0 |
| Kakaopulver | 30,0 | 30,0 |
| Saccharin | 0,5 | 0,5 |
| Verbindung der Formel (2) | 0,075 | 0,075 |

Zucker (bzw. vorgemischtes Lactitol und Saccharin) wird zusammen mit der Butter aufgeschlagen. Dann wir das Vollei hinzugegeben und ebenfalls untergerührt. Anschließend werden portionsweise das Mehl und das Kakaopulver untergemischt. Die Masse wird dann in eine Form gefüllt und für 60 Minuten bei 180 °C gebacken.

### ANWENDUNGSBEISPIEL 18

### Untersuchungen zur aktivierenden oder inhibierenden Wirkung der Verbindungen der Formeln (1) bis (4) auf T1R2/T1R3-Rezeptoren

Es wird analog zu Anwendungsbeispiel 3 vorgegangen um die konzentrationsabhängige Wirkung der Substanz auf Delta F/F darzustellen.

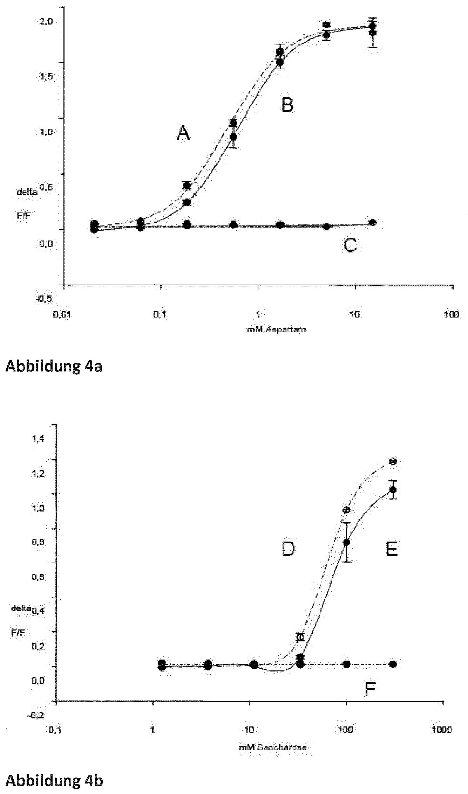

Die Ergebnisse sind in den **Abbildungen 4a und 4b** dargestellt. Gezeigt wird die Beeinflussung der durch Aspartam bzw. Sucrose verursachten Aktivierung des heterolog exprimierte Süßrezeptors TAS1R2/TAS1R3 durch die Lactisolderivate 2 und 3. A= Aspartam (EC₅₀ = 0,50 mM), B= Aspartam + 1 mM 2 (EC₅₀ = 0,61 mM), C= Aspartam + 1mM Lactisol, D = Saccharose (EC₅₀ = 65,7 mM), E = Saccharose + 1 mM 2 (EC₅₀ = 78,7 mM), F = Saccharose + 1 mM Lactisol, EC₅₀ = mittlere effektive Konzentration.

Aus dem Diagramm ist erkennbar, dass die erfindungsgemäße Verbindung 2 im Gegensatz zu Lactisol keinen antagonistischen Effekt in Gegenwart des beispielhaften süßen Stoffes Aspartam bzw. Sucrose zeigt und somit als selektiver Antagonisten des Süßrezeptors im Darm funktionieren können. Die Dosiswirkungskurve für Aspartam bzw. Saccharose wird durch die Gegenwart von 2 nicht verändert.

## Patentansprüche

1. Lactisolverbindungen der Formel (I) 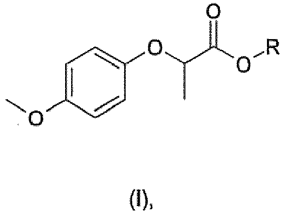 in der R für einen aromatischen oder aliphatischen Rest steht, der eine Gruppe -O-umfasst, die Bestandteil einer Hydroxy-, Ester- oder Ether-Gruppe ist, zur Verwendung als Arzneimittel zur Prävention und/oder Behandlung von Diabetes.

2. Verbindungen nach Anspruch 1, **dadurch gekennzeichnet, dass** die medizinische Indikation Diabetes Typ-2 ist.

3. Verbindungen nach Anspruch 1 und/oder 2, **dadurch gekennzeichnet, dass** die medizinische Indikation die orale Aufnahme vorsieht.

4. Verbindungen nach mindestens einem der Ansprüche 1 bis 3, **dadurch gekennzeichnet, dass** sie der Formel (II) folgen,
**R1O-CH₂-CH(OR2)-CH₂-OR3** **(II)**
wobei mindestens einer der Reste R1, R2 oder R3 eine Lactisolgruppe der Formel (I) darstellt und die gegebenenfalls weiteren Reste jeweils unabhängig voneinander für Wasserstoff oder einen Fettsäurerest stehen.

5. Verbindungen nach mindestens einem der Ansprüche 1 bis 4, **dadurch gekennzeichnet, dass** sie ausgewählt sind aus der Gruppe, die gebildet wird von
**(1)** 2-(4-Methoxyphenoxy)-propansäurehexylester;
**(2)** 2-(4-Methoxyphenoxy)propansaeure-2,3-bis-[2-(4-methoxyphenoxy)-propionyloxy]-propylester;
**(3)** 2-(4-Methoxyphenoxy)propansaeure-2-[2-(4-methoxyphenoxy)-propionyloxy]-ethxylester
**(4)** 2-(4-Methoxyphenoxy)propansäurebutylester
**(5)** 2-(4-Methoxyphenoxy)propansäure-(E)-hex-2-enylester und
**(6)** 2-(4-Methoxyphenoxy)-propansaeure-2-isopropyl-5-methyl-phenylester sowie deren Gemischen.

6. Zum Verzehr geeignete Zusammensetzung bestehend aus
(a) einer Lactisol-Verbindung der Formel (I) und
(b) mindestens einem weiteren zum Verzehr geeigneten Bestandteil.

7. Zusammensetzung nach Anspruch 6, **dadurch gekennzeichnet, dass** sie aus
(a) einer Lactisolverbindung der Formel (I) und
(b) festen Trägerstoffen
besteht, wobei das Gewichtsverhältnis der Gesamtmenge an Bestandteil (a) in der Zusammensetzung zur Gesamtmenge an festen Trägerstoffen (b) in der Zusammensetzung, jeweils bezogen auf die Trockenmasse der Zusammensetzung, im Bereich von 1:10 bis 1:100 000 liegt.

8. Zusammensetzung nach Anspruch 6, **dadurch gekennzeichnet, dass** sie aus
(a) einer Lactisolverbindung der Formel (I) und
(b) festen Trägerstoffen
besteht, wobei die Gesamtmenge an Bestandteil (a) und festen Trägerstoffen (b) in der Zusammensetzung, bezogen auf die Trockenmasse der Zusammensetzung, 70 bis 100 Gew.-% beträgt.

9. Zusammensetzung nach einem der Ansprüche 6 bis 8, **dadurch gekennzeichnet, dass** die Gesamtmenge an Bestandteil (a) bezogen auf das Gesamtgewicht der Zusammensetzung im Bereich von 0,5 ppm bis 5000 ppm liegt.

10. Zusammensetzung nach einem der Ansprüche 6 bis 9, **dadurch gekennzeichnet, dass** es sich um eine Halbfertigware handelt.

11. Verwendung von Lactisol-Verbindungen der Formel (I) als Süßrezeptor-Antagonisten.

12. Verwendung nach Anspruch 11, **dadurch gekennzeichnet, dass** die Süßrezeptor-Antagonisten T1R2/T1R3-Rezeptor-Antagonisten sind.

## Claims

1. Lactisol compounds of formula (I) 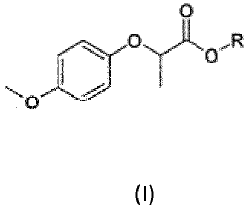 wherein R is an aromatic or aliphatic radical which comprises one --O-- group that is part of a hydroxy, an ester or an ether group, for use as a medicament for preventing and/or treating diabetes.

2. Compounds according to claim 1, **characterized in that** the medical indication is diabetes type 2.

3. Compounds according to claim 1 and/or 2, **characterized in that** the medical indication provides oral intake.

4. Compounds according to at least one of claims 1 to 3, **characterized in that** they follow formula (II),
**R¹O-CH₂-CH(OR²)-CH₂-OR³** **(II)**
wherein at least one of the radicals R1 to R3 is a lactisole group of formula (I) and any remaining radicals are, independently of one another hydrogen or fatty acid radicals

5. Compounds according to at least one of claims 1 to 4, **characterized in that** they are selected from the group consisting of
**(1)** 2-(4-methoxyphenoxy)propanoic acid hexyl ester;
**(2)** 2-(4-methoxyphenoxy)propanoicacid-2,3-bis-[2-(4-methoxyphenoxy)-propionyloxy]propyl ester;
**(3)** 2-(4-methoxyphenoxy)propanoic acid-2-[2-(4-methoxyphenoxy)-propionyloxy]-ethylester;
**(4)** 2-(4-methoxyphenoxy)propanoic butyl ester;
**(5)** 2-(4-methoxyphenoxy)propanoic acid-(E)-hex-2-enyl ester; and
**(6)** 2-(4-methoxyphenoxy)-propanoic acid-2-isopropyl-5-methyl-phenyl ester.

6. An edible composition consisting of
(a) a lactisol compound of formula (I) and
(b) at least one further edible component.

7. Composition according to claim 6, **characterized in that** the composition is consisting of
(a) a lactisol compound of formula (I) and
(b) solid carriers,
wherein the weight ratio of the total quantity of component (a) in the composition to the total quantity of solid carriers (b) in the composition, in relation to the dry weight of the composition, is in the range of 1:10 to 1:100,000.

8. Composition according to claim 6, **characterized in that** the composition is consisting of
(a) a lactisol compound of formula (I) and
(b) solid carriers,
wherein the total quantity of component (a) and solid carriers (b) in the composition, in relation to the dry weight of the composition, is 70 to 100 weight %.

9. Composition according to any of claims 6 to 8, **characterized in that** the total quantity of component (a) in relation to the total weight of the composition is in the range of 0.5 ppm to 5,000 ppm.

10. Composition according to any of claims 6 to 9, **characterized in that** the composition is a semi-finished product.

11. Use of lactisol compounds of formula (I) as sweet taste receptor antagonists.

12. Use according to claim 11, **characterized in that** the sweet taste receptor antagonists are T1R2/T1R3 receptor antagonists.

## Revendications

1. Composés de lactisol de formule (I) 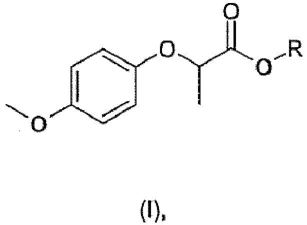 dans laquelle R représente un radical aromatique ou aliphatique, qui comprend un groupe -O- qui fait partie d'un groupe hydroxy, ester ou éther, destinés à une utilisation en tant que médicament pour la prévention et/ou le traitement du diabète.

2. Composés selon la revendication 1, **caractérisés en ce que** l'indication médicale est le diabète de type 2.

3. Composés selon la revendication 1 et/ou 2, **caractérisés en ce que** l'indication médicale prévoit l'administration par voie orale.

4. Composés selon au moins l'une quelconque des revendications 1 à 3, **caractérisés en ce qu'**ils suivent la formule (II)
**R¹O-CH₂-CH(OR²)-CH₂-OR³** **(II)**
dans laquelle au moins un des radicaux R1, R2 ou R3 représente un groupe lactisol de formule (I) et les autres radicaux éventuels représentent chacun indépendamment les uns des autres l'hydrogène ou un radical acide gras.

5. Composés selon au moins l'une quelconque des revendications 1 à 4, **caractérisés en ce qu'**ils sont choisis dans le groupe formé par :
**(1)** l'ester hexylique de l'acide 2-(4-méthoxyphénoxy)-propanoïque ;
**(2)** l'ester 2,3-bis-[2-(4-méthoxyphénoxy)-propionyloxy)-propylique de l'acide 2-(4-méthoxyphénoxy)-propanoïque ;
**(3)** l'ester 2-[2-(4-méthoxyphénoxy)-propionyloxy)-éthylique de l'acide 2-(4-méthoxyphénoxy)-propanoïque ;
**(4)** l'ester butylique de l'acide 2-(4-méthoxyphénoxy)-propanoïque ;
**(5)** l'ester (E)-hex-2-énylique de l'acide 2-(4-méthoxyphénoxy)-propanoïque ; et
**(6)** l'ester 2-isopropyl-5-méthyl-phénylique de l'acide 2-(4-méthoxyphénoxy)-propanoïque,
ainsi que leurs mélanges.

6. Composition apte à la consommation, constituée par :
(a) un composé de lactisol de formule (I) et
(b) au moins un autre constituant apte à la consommation.

7. Composition selon la revendication 6, **caractérisée en ce qu'**elle est constituée par :
(a) un composé de lactisol de formule (I) et
(b) des véhicules solides,
le rapport en poids entre la quantité totale de constituant (a) dans la composition et la quantité totale de véhicules solides (b) dans la composition, à chaque fois par rapport à la masse sèche de la composition, se situant dans la plage allant de 1:10 à 1: 100,000.

8. Composition selon la revendication 6, **caractérisée en ce qu'**elle est constituée par :
(a) un composé de lactisol de formule (I) et
(b) des véhicules solides,
la quantité totale de constituant (a) et de véhicules solides (b) dans la composition, par rapport à la masse sèche de la composition, étant de 70 à 100 % en poids.

9. Composition selon l'une quelconque des revendications 6 à 8, **caractérisée en ce que** la quantité totale de constituant (a), par rapport au poids total de la composition, se situe dans la plage allant de 0,5 ppm à 5 000 ppm.

10. Composition selon l'une quelconque des revendications 6 à 9, **caractérisée en ce qu'**il s'agit d'un produit semi-fini.

11. Utilisation de composés de lactisol de formule (I) en tant qu'antagonistes des récepteurs du goût sucré.

12. Utilisation selon la revendication 11, **caractérisée en ce que** les antagonistes des récepteurs du goût sucré sont des antagonistes des récepteurs T1R2/T1R3.
